# FASCICULE DE BREVET EUROPEEN

(11) **EP 2 167 639 B1**
(45) Date de publication et mention de la délivrance du brevet: **17.08.2016**
(21) Numéro de dépôt: 08806147.8
(22) Date de dépôt: 01.07.2008
(51) Int. Cl.: C12N 1/20, C12R 1/21

(54) **MILIEU DE CULTURE POUR HAEMOPHILUS INFLUENZAE DE TYPE B**
KULTURMEDIUM FÜR HAEMOPHILUS-INFLUENZAE VOM TYP B
CULTURE MEDIUM FOR HAEMOPHILUS INFLUENZAE TYPE B

(30) Priorité: 10.07.2007 FR 0756371
(43) Date de publication de la demande: 31.03.2010
(73) Titulaire: Sanofi Pasteur, 69367 Lyon Cedex 07 (FR)
(72) Inventeur: MAITRE-WILMOTTE, Ghislaine, F-69003 Lyon (FR); ROKBI, Bachra, F-69003 Lyon (FR); SPECK, Denis, F-69110 Sainte Foy Les Lyon (FR)
(86) Numéro de dépôt international: PCT/FR2008/051223
(87) Numéro de publication internationale: WO 2009/007641

(56) Documents cités:
- WO-A-97/00697
- US-A- 4 459 286
- TAKAGI MICKIE ET AL: "Improved cultivation conditions for polysaccharide production by H-influenzae type b" JOURNAL OF CHEMICAL TECHNOLOGY AND BIOTECHNOLOGY, vol. 81, no. 2, février 2006 (2006-02), pages 182-188, XP002469212 ISSN: 0268-2575
- DATABASE BIOSIS [Online] BIOSCIENCES INFORMATION SERVICE, PHILADELPHIA, PA, US; 2003, DAINES D A ET AL: "A mutation in ydgQ affects growth and haemolysis of Haemophilus influenzae." XP002506858 Database accession no. PREV200300520545 & ABSTRACTS OF THE GENERAL MEETING OF THE AMERICAN SOCIETY FOR MICROBIOLOGY, vol. 103, 2003, pages D-091 URL, 103RD AMERICAN SOCIETY FOR MICROBIOLOGY GENERAL MEETING; WASHINGTON, DC, USA; MAY 18-22, 2003 ISSN: 1060-2011
- MERTEN O-W ET AL: "THE NEW MEDIUM MDSS2N, FREE OF ANY ANIMAL PROTEIN SUPPORTS CELL GROWTH AND PRODUCTION OF VARIOUS VIRUSES" CYTOTECHNOLOGY, KLUWER ACADEMIC PUBLISHERS, DORDRECHT, NL, vol. 30, no. 1-3, 1999, pages 191-201, XP000978974 ISSN: 0920-9069

## Description

L'invention a pour objet un milieu de culture *d'Haemohilus influenzae type b,* dans lequel la source d'azote protéique comprend au moins une peptone végétale et dans lequel la source d'hème est constituée par de la protoporphyrine IX. L'invention concerne également un procédé de production de Polyribosyl ribitol phosphate (PRP) utilisé dans la fabrication d'un vaccin contre la méningite à *Haemophilus influenzae de type b.*

La capsule est le facteur majeur de virulence des souches *d'Haemophilus influenzae de type b*. C'est un polysaccharide formé de l'enchaînement d'unités répétitives de ribosylribitol phosphate. On emploie indifféremment l'expression polyribosylribitol phosphate (PRP) ou polysaccharide capsulaire de type b pour désigner la capsule *d'Haemophilus influenzae de type b.*

Les populations *d'Haemophilus influenzae type b* sont souvent hétérogènes ; des bactéries capsulées coexistent avec des bactéries non capsulées. Les bactéries non capsulées perdent leur capacité à exprimer la capsule à la suite de mutations génétiques qui se produisent spontanément. D'après Hoiseth et al. (Infectious and Immunity (1985), 49 : 389-395), la perte de l'expression de la capsule se produit à une fréquence de 0,1 à 0,3% à chaque génération bactérienne. Sur le plan génétique, il a été montré que le locus *cap* intervient dans l'expression de la capsule à la surface de ces bactéries (Kroll, J. S., et coll. J. Bacteriol. (1988) 170: 859-864). Les bactéries capsulées ont un locus *cap* qui contient au moins deux copies d'un gène de 18Kb. Les bactéries non capsulées n'ont plus de gène de 18 Kb ou seulement une seule copie de ce gène. Pour identifier les bactéries capsulées on utilise habituellement le test d'agglutination sur lame des bactéries en présence d'anticorps anti PRP ou on met en oeuvre des techniques de biologie moléculaire caractérisant le locus *cap.*

Les vaccins à base de PRP, ou de PRP couplé par liaison de covalence à une protéine porteuse sont utilisés pour prévenir les infections à *Haemophilus influenzae de type b.* Pour fabriquer ces vaccins il faut produire de grandes quantités de bactéries dans des volumes importants de milieu de culture dont on extrait le PRP que l'on purifie ensuite. Néanmoins, la facilité qu'ont les bactéries capsulées *d'Haemophilus influenzae de type b* à réverter vers des formes non capsulées peut constituer un écueil pour la production de PRP.

Pour la production industrielle de PRP, on utilise classiquement des milieux de culture à base de peptones animales qui représentent la source principale d'azote protéique supplémentés en extrait de levure, glucose, hémine, β NAD et sels minéraux. A titre d'exemple, on cite le milieu de production décrit dans US 4,459,286.

En raison des risques liés à la BSE on cherche à remplacer les produits d'origine animale et plus particulièrement les produits d'origine humaine ou bovine par des produits offrant une meilleure sécurité biologique.

Carty et coll (in Dev. Indust. Microbiol. 26 : 763-767 (1985)) ont montré que les peptones animales pouvaient être remplacées par de la peptone de soja, pour la production de PRP. La composition par litre de ce milieu (milieu MP) est la suivante : Peptone de soja : 10g ; Extrait de levure : 10 ml ; NaCl : 5g ; K₂HPO₄ : 2,5g ; Na₂HPO₄ : 3,3g ; dextrose : 5g ; Chlorure d'hémine : 10mg ; NAD : 10mg.

Takagi et coll (J. Chem. Tech. and Biotech 81 : 182-188 (2006) ont cherché à optimiser la composition du milieu de Carty (milieu MP). Ils ont montré que la concentration en PRP dans le milieu de culture était augmentée de 70% pour atteindre 0,25 g/L lorsque la concentration en hémine et en β NAD était augmentée. Pour augmenter la production en PRP il apparait donc comme nécessaire d'augmenter les concentrations en cofacteurs (hémine et βNAD) qui sont nécessaires à la croissance *d 'Haemophilus influenzae type b.*

Il existe donc toujours un besoin d'améliorer les procédés de production de PRP notamment lorsque les volumes de culture sont importants (≥100 litres) tout en appliquant les meilleures conditions de sécurité biologique.

C'est pourquoi, l'invention a pour objet un nouveau milieu de culture d'*Haemophilus influenzae type b* caractérisé en ce que la source d'azote protéique est d'origine non animale et comprend au moins une peptone végétale et en ce que la source d'hème est constituée par de la protoporphyrine IX. Un tel milieu convient particulièrement pour la production industrielle de PRP. Il offre une plus grande sécurité biologique puisque les peptones d'origine animale ont été remplacées par des peptones végétales. Il répond également à des contingences de coût de production puisqu'il faut 10 à 20 fois moins de protoporphyrine IX que d'hémine pour obtenir des taux de PRP dans les surnageants de culture qui soient exploitables sur le plan industriel (taux ≥ 0,2g/L).

Par « milieu pour la culture d'*Haemophilus influenzae de sérotype b,* on entend un milieu favorisant la croissance d'*Haemophilus influenzae de sérotype b* et qui comprend:
- une source d'azote protéique,
- une source d'hème,
- une source de β NAD,
- une source de carbohydrate,
- une source de vitamines et de facteurs de croissance, et
- des sels minéraux.

Par « source d'azote protéique » on entend une préparation dans laquelle la quantité d'acides aminés, de peptides, de polypeptides, de peptones et/ou de protéines représentent au moins 50% du poids sec de cette préparation.

Par « source d'azote protéique d'origine non animale » on entend une source d'azote protéique qui a une origine non animale. Par conséquent, la préparation n'est pas produite à partir de cellules animales, de tissus animaux, d'organes ou d'organismes animaux. Elle est généralement produite à partir de végétaux, d'algues, de bactéries, de levures ou de champignons.

Le milieu de culture selon l'invention désigne en premier lieu un milieu liquide mais il peut aussi se présenter sous forme solide. La forme solide est obtenue par addition au milieu liquide d'une substance gélifiante, telle que l'agar que l'on utilise habituellement dans une gamme de concentration allant de 10 à 30g/l.

La source d'hème, selon l'invention est représentée par de la protoporphyrine IX de formule :

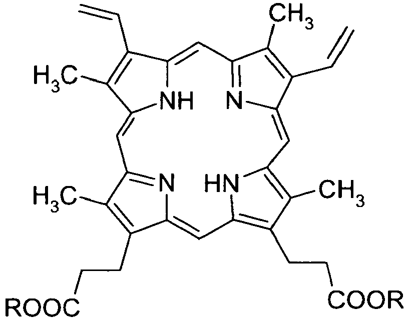

Où R désigne indifféremment H, ou un sel, de façon préférée un sel de métal alcalin, en particulier un sel de sodium.

La protoporphyrine IX dans le cas de la présente invention est non complexée au fer. Jusqu'à présent tous les milieux préconisés pour produire le PRP contenaient comme source d'hème soit une protoporphyrine IX complexée avec du fer (il s'agit de l'hème), soit une protoporphyrine IX complexée avec FeCl (il s'agit de l'hémine), soit enfin et plus rarement une protoporphyrine IX complexée avec FeOH (il s'agit de l'hématine). Même si les souches d'Haemophilus influenzae serotype b possèdent une ferrochelatase qui leur permet de transformer la protoporphyrine IX, en une forme complexée avec du fer (Loeb et coll, J. Bacteriology (1995), 177 ; 3613-3615), il n'a jamais été montré que l'on pouvait utiliser comme source d'hème de la protopophyrine IX non complexée dans un milieu de culture à base de peptone végétale pour produire du PRP à une concentration qui soit exploitable sur le plan industriel. On considère généralement qu'il faut un taux de PRP d'au moins 0,1 à 0,2 g/l dans le surnageant de culture pour assurer une production industrielle de PRP.

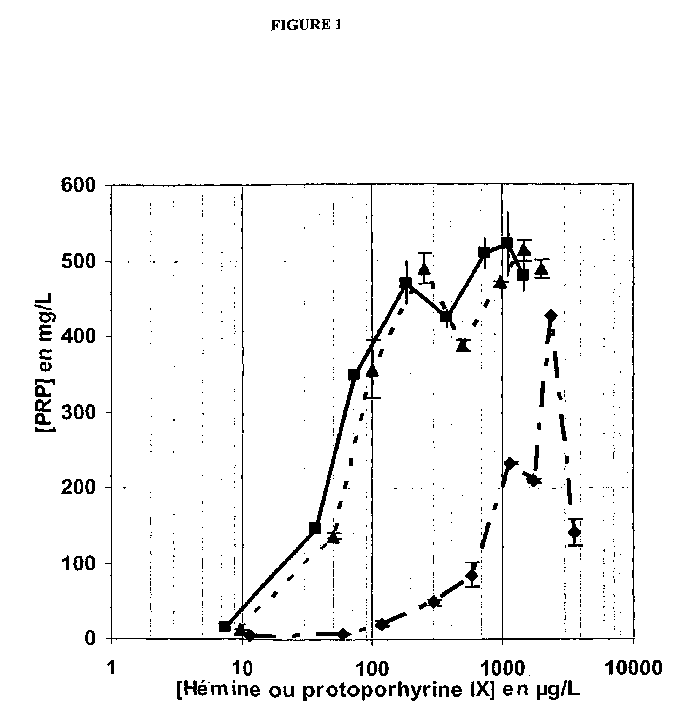

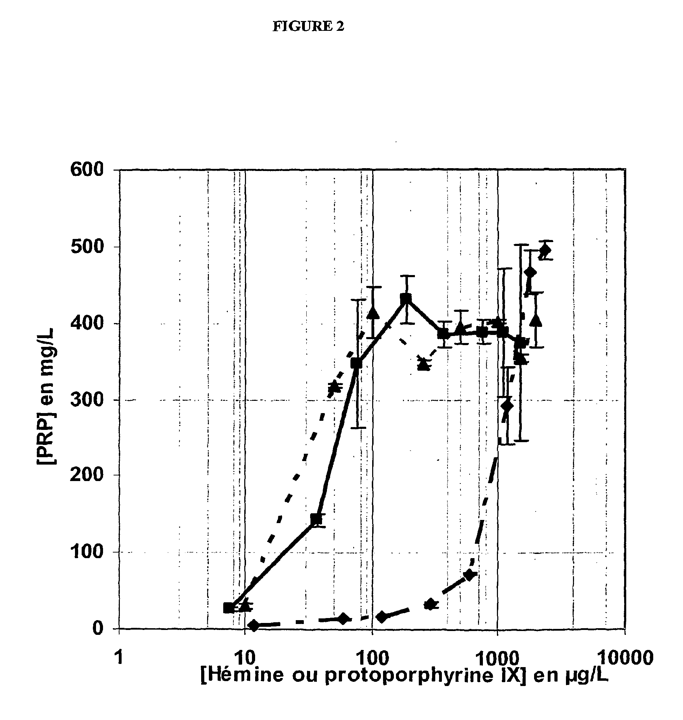

De façon surprenante on a remarqué qu'en utilisant comme source d'hème une protoporphyrine IX et comme source d'azote protéique une peptone végétale, les concentrations en protoporphyrine IX nécessaires étaient 10 à 100 fois plus faibles que celles qui sont utilisées lorsque la source d'hème est une protoporphyrine IX complexée au fer. Comme le montre l'exemple 1, il suffit d'une concentration en protoporphyrine IX comprise entre 100 et 200µg/l pour obtenir une production maximale en PRP (la concentration dans le surnageant de culture est ≈0,4g/l) alors qu'il faut une concentration 10 à 20 fois plus forte en hémine pour obtenir une production équivalente en PRP. Par ailleurs, à une concentration aussi faible que 10µg/l de protoporphyrine IX on observe déjà une production significative en PRP alors qu'elle est insignifiante avec de l'hémine. A 50µg/l de protoporphyrine IX, la production en PRP atteint ou dépasse 100pg/l (ce qui est déjà un taux utilisable à l'échelle industrielle) tandis qu'elle est de l'ordre de 10µg/l voire moins lorsque le milieu de culture renferme de l'hémine à la même concentration. (voir figures 1 et 2).

C'est pourquoi l'invention a pour objet :
Un milieu de culture selon l'invention dans lequel la concentration en protoporphyrine IX est d'au moins 0,01 mg/l, d'au moins 0,02mg/l, d'au moins 0,03mg/l, d'au moins 0,04mg/l ou de façon préférée d'au moins 0,05mg/l.
Généralement, la concentration en protoporphyrine IX dans le milieu de culture est entre 0,1 mg/l et 5mg/l et de façon préférée entre 0,1 mg/l et 2 mg/l. Dans ces gammes de concentration il y a une utilisation optimale des matières premières pour une production optimale de PRP dans les surnageants de culture.

La protoporphyrine IX convenant à l'objet de l'invention peut être d'origine animale et produite à partir de tissus animaux (bovins, porcins,...). Le degré de pureté de ces préparations est généralement d'au moins 80%, de façon préférée d'au moins 90%, et de façon encore plus préférée d'au moins 95% (poids/poids). Bien que les contaminants puissent contenir des quantités résiduelles d'acides aminés, de peptides et ou de protéines, les préparations de protoporphyrine IX ne peuvent pas être considérées comme une source d'azote protéique au sens de l'invention dans la mesure où les quantités résiduelles d'acides aminés, de peptides et /ou de protéines, éventuellement présentes représentent généralement moins de 5%, en général moins de 1% du poids sec des préparations.

De façon préférée, pour garantir une plus grande sécurité biologique on utilise une protoporphyrine IX qui soit exempte de tout contaminant d'origine animale. Pour produire une telle protoporphyrine IX on peut utiliser le procédé de production tel que décrit dans la demande de brevet française enregistrée sous le n° d'enregistrement 07/02334 et déposé le 30/03/2007 en utilisant les étapes qui sont décrites dans le schéma 2.

Dans un autre mode de réalisation préférée, le milieu de culture selon l'invention comprend une protoporphyrine IX exempte de tout contaminant d'origine animale.

Selon l'objet de l'invention, la source principale d'azote protéique est représentée par une ou plusieurs peptones végétales. Elles sont généralement sous la forme d'hydrolysats. Ils sont obtenus par traitement enzymatique ou chimique des protéines extraites généralement des parties de la plante qui ont les plus fortes teneurs en protéines. De façon préférée, on utilise des plantes qui n'ont pas été génétiquement modifiées. Lorsque l'on utilise la voie chimique, une des méthodes consiste à traiter l'extrait protéique avec de l'acide chlorhydrique à chaud et sous pression. L'hydrolysat est ensuite neutralisé par de la soude puis débarrassé des sous produits solides. Lorsqu'on utilise la voie enzymatique, une des méthodes classiques consiste à traiter l'extrait protéique avec de la papaïne.

Les peptones végétales sont des préparations contenant principalement un mélange d'acides aminés, et de petits peptides dont le PM est ≤1 KD. Les peptides dont le PM est > 1 KD représentent généralement moins de 40 % du mélange. On peut également au besoin utiliser des hydrolysats ultrafiltrés pour enrichir ou sélectionner les peptides de faible taille. On peut également soumettre l'hydrolysat ultrafiltré à une étape de chromatographie supplémentaire pour sélectionner des fractions d'hydrolysats ayant un PM ≤ 1 KD, ou ≤500 Daltons, ou même ≤350 Daltons. On obtient ainsi des préparations de peptones végétales dans lesquelles plus de 40% des peptides, plus de 50% des peptides, ou même plus de 60% des peptides ont un PM ≤ 1KD, ou ≤500 Daltons, ou même ≤350 Daltons. Les peptones végétales convenant à l'objet de l'invention, proviennent notamment de la pomme de terre comme celles fournies par Organotechnie (plant peptone E1 ou plant peptone ET1), du soja comme celles fournies par Organotechnie ou Kerry, du coton (Hy cotton fournie par Quest), du riz (Hy rice fournie par Kerry), de la fève fournie par Solabia, du blé comme celles fournies par Oganotechnie (wheat peptone E1) ou Kerry (Hypep ™ 4602 , Hypep ™ 4601) ou du petit pois, notamment les hydrolysats enzymatiques du petit pois fournis par Kerry (HY pea 7404) ou oxoid (VG 100) ou des hydrolysats acides du petit pois fournis par Oxoïd référencé sous l'appellation « Acid hydrolysed vegetable peptone ». De façon préférée, la peptone végétale convenant à l'objet de l'invention est une peptone de blé et de façon encore plus préférée la peptone végétale est une peptone de petit pois.

Pour définir les concentrations d'utilisation de la peptone végétale on tient compte de la teneur en azote protéique de la peptone. Cette teneur est calculée en utilisant la méthode de Kjeldahl (Lynch JM et coll., J AOAC Int. (1999) 82(6):1389-98). Habituellement, les teneurs en azote protéique des peptones végétales convenant à l'objet de l'invention sont comprises entre 8 % et 15% par gramme de peptone (poids/poids). Dans cette gamme, on obtient de bons résultats lorsque la concentration en peptone végétale dans un milieu de culture selon l'invention correspond à une concentration en azote protéique allant de 0,08 à 2,25 g/l et façon préférée dans une gamme de concentration allant de 0,4 à 1,5g/l.

C'est pourquoi l'invention a pour objet un milieu dans lequel la concentration totale en peptone végétale équivaut à une concentration en azote protéique allant de 0,08g/l à 2,25g/l.

Comme source de β NAD (encore appelé facteur V ou β nicotinamide adenine dinucléotide), on utilise une préparation purifiée de β NAD lui-même, ou une préparation purifiée contenant un dérivé du β NAD choisi parmi le nicotinamide riboside (NR), le β nicotinamide adenine mononucléotide (NMN), ou le β nicotinamide adénine dinucléotide phosphate (NADP). Le degré de pureté de la préparation est généralement d'au moins 80%, de façon préférée d'au moins 90% et de façon encore plus préférée d'au moins 95%. On utilise de préférence dans le cas de la présente invention une source de β NAD exempte de contaminant protéique d'origine animale. Ces préparations purifiées sont utilisées à une concentration d'au moins 1µM. A titre d'exemple, on utilise du β NAD à une concentration allant de 2 à 50 mg/l de milieu de culture.

Comme source de carbohydrate on peut utiliser tout sucre qui est métabolisé par *Haemophilus influenzae type b,* comme le fructose, le ribose, le xylose, le fucose, le glycerol, ou plus particulièrement le glucose. Généralement, la source de carbohydrate a une origine non animale et la concentration en carbohydrate dans le milieu de culture est d'au moins 10 mM. Lorsque le glucose est utilisé, sa concentration dans le milieu de culture est généralement comprise entre 2 à 20 g/l.

Le milieu de culture selon l'invention comprend également une source de vitamines et de facteurs de croissance. On utilise à cette fin un extrait de levure qui provient de la fraction soluble du produit d'autolyse de la levure de bière issue de la culture de *Saccharomyces sp.* On retrouve dans sa composition de nombreux acides aminés, des vitamines telles que les vitamines B5, B1, B2, B6, PP, H et B12, des oligo éléments, des dérivés oligo nucléotidiques. Les extraits autolytiques de levure commercialement disponibles produits par Quest, Difco ou Solabia conviennent à l'objet de l'invention.

La concentration de l'extrait de levure dans le milieu selon l'invention se situe habituellement dans une gamme de concentration allant de 0,2g/l à 15g/l, préférentiellement dans une gamme de concentration allant de 0,2 g/l à 10 g/l et de façon encore plus avantageuse dans une gamme de concentration allant de 0,2 à 5 g/l. On a observé que la production en PRP par les bactéries était meilleure lorsque la concentration en extrait de levure se situait dans la gamme de concentration allant de 0,2 à 5g/L.

L'extrait de levure représente également une source accessoire d'azote protéique d'origine non animale. Les teneurs en azote protéique dans les extraits de levure sont en effet généralement de 9 à 11% (poids/poids). Afin d'éviter un hyper catabolisme azoté pouvant être responsable de l'accumulation de déchets toxiques au cours de la culture, on ajuste généralement les concentrations en peptone(s) végétale(s) et en extrait de levure de telle sorte que la teneur en azote protéique totale dans le milieu ne dépasse pas 2,5 g/l. De façon préférée, on ajuste les concentrations en peptone(s) végétale(s) et en extrait de levure de telle sorte que la teneur en azote protéique totale dans le milieu selon l'invention soit de 0,5 à 2,5 g/l.

Le milieu de culture selon l'invention comprend également des sels minéraux. Les sels minéraux utilisés sont généralement sous forme de solutions salines, dont au moins une exerce un pouvoir tampon suffisant pour que le pH initial du milieu, avant ensemencement des bactéries, se situe de façon préférée entre 6,5 et 7,5 et de façon particulièrement préférée entre 7 et 7,5. Généralement on utilise un mélange de cations monovalents tels que Na⁺ et/ou K⁺, de cations divalents tels que Ca⁺⁺ et/ou Mg⁺⁺, d'anions phosphate sous la forme HPO₄⁻⁻, H₂PO4⁻ et/ou PO4⁻⁻ et d'anions SO₄⁻⁻, et Cl⁻ sous forme de solutions salines dont les molarités peuvent varier habituellement dans une gamme de concentration allant de 10⁻² mM à 100 mM.

Outre les composants décrits dans les paragraphes précédents, il est bien entendu qu'un milieu de culture selon l'invention peut incorporer dans sa composition, un ou plusieurs autres composants minéraux et/ou organiques pourvus qu'ils n'interfèrent pas négativement dans la production de PRP. De façon très préférée, on introduit des composants provenant d'une source non animale. On peut ainsi ajouter au milieu selon l'invention des acides aminés produits par synthèse chimique, ou par fermentation microbiologique tels que le tryptophane et/ou la cystine, de l'azote minéral généralement sous forme de solutions salines apportant des ions NH₄⁺ et /ou même d'autres substances comme le lactate de sodium. Ces additifs sont généralement utilisés à des concentrations faibles. Le complément en acides aminés dans le milieu de culture est généralement à une concentration ≤ 1 mM. De même, les sels d'ammonium et/ou le lactate de sodium sont généralement à une concentration ≤ 10 mM. Enfin, bien qu'il ne soit pas nécessaire de compléter le milieu de culture selon l'invention par un apport de fer sous forme de fer ionique puisqu'il est déjà présent en quantité suffisante dans la composition de peptone végétale et d'extrait de levure, on peut par précaution ajouter au milieu de culture une solution de sel de fer, dans une gamme de concentration pouvant aller de 0,5 à 10 mg/l pour prévenir tout déficit en fer qui pourrait subvenir au cours de la croissance bactérienne.

Avantageusement, le milieu de culture selon l'invention est exempt de toute protéine, de tout polypeptide, peptide et/ou acide aminé d'origine humaine ou bovine ou même exempt de toute protéine, de tout polypeptide, peptide et/ou acide aminé d'origine animale, ou encore plus avantageusement exempt de tout contaminant d'origine animale.

Selon un mode de réalisation particulier, l'invention a pour objet un milieu de culture liquide qui comprend:
- de 0,1 mg/l à 5 mg/l de protoporphyrine IX,
- de 2 à 50 mg/l de β NAD,
- de 2 à 20 g/l de glucose,
- de 2 à 5 g/l d'un extrait de levure,
- une peptone de petit pois équivalent à une concentration en azote protéique de 0,4 g/l à 1,5 g/l, et
- un cocktail d'ions minéraux comprenant les ions Na⁺, NH4 ⁺, Ca⁺⁺, Mg⁺⁺, HPO₄⁻⁻ H₂PO4⁻, SO₄^{- -}, et Cl⁻ sous forme de solutions salines de sorte que le pH du milieu se situe entre 6,5 et 7,5, de façon préférée entre 7,0 et 7,5.

En utilisant cette composition de milieu, on empêche le développement de bactéries révertantes non capsulées pendant la culture en milieu liquide. On a remarqué en effet, qu'en ensemençant dans ce milieu une population qui renferme initialement 100% de bactéries capsulées (le génome de toute la population bactérienne a un locus *cap* qui contient deux copies du gène de 18Kb), on obtient après une période de culture équivalente à 40 générations bactériennes, une population bactérienne qui renferme toujours 100% de bactéries capsulées. Cette composition de milieu, qui inclut en particulier celle qui est citée dans l'exemple 3.2.2.1 et qui est utilisée dans l'exemple 4, contribue à améliorer les rendements en PRP en exerçant un rôle stabilisant sur la population des bactéries capsulées (cf. exemple 4).

Selon un autre aspect, l'invention a pour objet un procédé de production du polyribosylribitol phosphate (PRP) dans lequel :
(i) on cultive *Haemophilus influenzae de sérotype b* dans un milieu de culture selon l'invention,
(ii) on prélève le surnageant de la culture obtenue en (i), et
(iii) on extrait le PRP du surnageant de culture.

Pour produire le PRP selon le procédé de l'invention, on peut procéder dans l'étape (i) à une ou plusieurs cultures successives d'*Haemophilus influenzae de serotype b* dans un milieu liquide selon l'invention. Les cultures successives permettent d'augmenter la biomasse.

Pour ce faire, les bactéries provenant d'un lyophilisat ou d'un congelât sont ensemencées dans un volume de milieu n'excédant généralement pas 1 litre. Au bout d'une nuit de culture ou lorsque la densité optique du milieu est suffisante, on transfère cette première culture dans un second milieu de culture identique au premier, mais dont le volume peut être jusqu'à 10 à 20 fois plus grand. La quantité de bactéries ensemencées dans le second milieu est ajustée de telle sorte que la densité optique (D.O.) initiale du second milieu de culture à 600 nm soit comprise entre 0,2 et 0,4 pour favoriser une croissance rapide de la population bactérienne. Cette deuxième culture est habituellement réalisée en fermenteur mais d'autres types de récipients peuvent être utilisés (flasks, spinners,...). Lorsque la culture est effectuée en fermenteur, on utilise habituellement pendant la durée de la culture une température de 37 °C +/- 1°C, une agitation constante, une pression de 0,1 bar, une pO2 de 30% et un débit d'air de 0,25 volume de gaz par volume de milieu par minute. Il est de la compétence de l'homme de l'art de choisir d'autres paramètres pour ce type de culture. A la fin de la phase exponentielle de croissance bactérienne, on peut encore amplifier la biomasse en la transférant dans un autre fermenteur de plus grande capacité en utilisant la même procédure et ainsi de suite. Les volumes de culture obtenus peuvent atteindre, voire dépasser 1000 litres. La(les) culture(s) est (sont) généralement réalisée(s) selon le mode « Batch ». Il est également possible d'adopter d'autres modes de culture, notamment le mode « feed batch ». Dans ce cas on rajoute au milieu un complément nutritif en carbohydrate pendant la phase exponentielle de croissance de façon à prolonger la multiplication bactérienne et à obtenir en fin de phase exponentielle de croissance une densité bactérienne plus importante. La quantité de carbohydrate ajoutée est évaluée en fonction du taux de lactate présent dans le milieu au moment de l'ajout.

On prélève finalement le surnageant de la dernière culture après inactivation des bactéries. L'inactivation est classiquement réalisée à l'aide d'une solution de formol à une concentration finale de 0,35%-0,37% (V/V). Le surnageant est classiquement séparé des bactéries par une étape de centrifugation. Le PRP contenu dans le surnageant résultant est ensuite extrait et purifié selon des procédés classiques bien connus de l'homme du métier.

Selon un autre mode de réalisation, l'invention a pour objet un procédé de production de PRP dans lequel :
(i) on cultive *Haemophilus influenzae de sérotype b* sur un milieu solide,
(ii) on transfère et on cultive une ou plusieurs colonies obtenues en (i) dans un milieu de culture selon l'invention,
(iii) on prélève le surnageant de la culture obtenue en (ii), et
(iv) on extrait le PRP du surnageant de culture.

Le milieu de culture solide utilisable dans le procédé de l'invention doit convenir à la culture d'*Haemophilus influenzae serotype b.* Il comprend également :
- une source d'azote protéique,
- une source d'hème,
- une source de β NAD,
- une source de carbohydrate,
- une source de vitamines et de facteurs de croissance,
- des sels minéraux, et
- une substance gélifiante, habituellement de l'agar à une concentration de 10 à 30 g/l.

Dans le procédé de production industrielle de PRP on utilise classiquement une étape préliminaire de culture en milieu solide. Habituellement les bactéries provenant d'un lyophilisat ou d'un congelât sont remises en suspension puis ensemencées sur un milieu solide à base de charbon complémenté en sang de cheval. Au bout de 16 à 20 heures de culture dans un incubateur à 37°C sous 10% de CO2, on prélève des colonies bactériennes que l'on amplifie en milieu liquide. Ce procédé présente l'inconvénient d'utiliser un milieu solide qui contient comme source d'azote proteique des protéines d'origine animale. Les inventeurs ont donc cherché à identifier des compositions de milieux solides dans lesquels la source d'azote protéique est dépourvue de toute protéine d'origine animale.

Initialement, les inventeurs ont mis en évidence qu'il était possible d'utiliser un milieu solide dans lequel l'extrait de levure pouvait servir à la fois de source d'azote protéique et de source de vitamines et de facteurs de croissance, les sources d'hème, de β NAD, de carbohydrate, et de sels minéraux ayant les mêmes caractéristiques que celles qui ont été précédemment décrites. Une concentration minimale de 0,05 mg/L de protoporhyrine IX, de 0,1 µM pour la source de β NAD et de 0,1 mM pour la source de carbohydrate sont préconisées tandis que la concentration en extrait de levure dans le milieu correspond à une teneur en azote protéique de 0,2 à 1,5 g/l. Les colonies obtenues sont viables, même si la croissance symbolisée par la taille des colonies n'est pas toujours optimale. Elles peuvent être transférées directement dans un milieu de culture liquide selon l'invention. On procède ensuite comme précédemment pour amplifier les volumes de culture, extraire et purifier le PRP.

De façon préférée, le milieu de culture solide comprend comme source d'azote protéique au moins une peptone d'origine végétale utilisée sous la forme d'un hydrolysat chimique ou enzymatique. On peut utiliser notamment, en complément de l'extrait de levure, au moins une peptone végétale provenant du blé, du coton, du riz, du soja, de féverolle, de pomme de terre, de petit pois, ou un mélange de celles-ci à une concentration en azote protéique pouvant aller notamment de 0,02 g/l à 2 g/l, la concentration en azote protéique totale dans le milieu solide n'excédant pas de préférence 2,5 g/l. Comme exemple de mélanges de peptones végétales que l'on peut utiliser on cite un mélange à base de peptones de soja, de coton et de riz ou un mélange à base de peptones de petit pois, de coton et de blé ou enfin un mélange à base de petit pois et de pomme de terre. Les inventeurs ont en effet noté que lorsque le milieu solide comprend également une peptone végétale comme source d'azote protéique la croissance bactérienne et la viabilité des bactéries étaient meilleures que celles observées sur un milieu solide à base de charbon (« charbon agar») additionné de sang cuit et défibriné de cheval. Elles sont maximales lorsque la peptone végétale utilisée est une peptone de petit pois.

C'est pourquoi l'invention a également pour objet un procédé de production de PRP dans lequel la source d'azote protéique du milieu solide est exempte de protéine d'origine animale et comprend au moins une peptone végétale. De façon préférée la peptone végétale est une peptone de petit pois.

Avantageusement, le milieu de culture solide selon l'invention est exempt de toute protéine, de tout polypeptide, de tout peptide et/ou de tout acide aminé d'origine humaine ou bovine ou encore plus avantageusement exempt de tout contaminant d'origine animale. Dans ce dernier cas, la source d'hème est constituée par de la protoporphyrine IX synthétique, la source de β NAD et de carbohydrate étant également d'origine non animale, la source de vitamines et de facteurs de croissance étant fourni par un extrait de levure et la substance gélifiante étant de l'agar (un produit dérivé des algues).

C'est pourquoi l'invention a également pour objet un procédé de production de PRP selon lequel le milieu de culture solide et le milieu de culture liquide sont dépourvus de tout contaminant d'origine animale.

On a cherché également à optimiser la composition du milieu solide de sorte qu'il soit possible de sélectionner les colonies bactériennes qui produisent le plus de PRP. La composition d'un tel milieu doit permettre d'obtenir :
- une bonne individualisation des colonies ;
- une bonne viabilité des colonies ;
- un développement et une taille suffisante des colonies pour pouvoir étudier leur morphologie. Pour pouvoir discriminer les colonies il faut un milieu qui permette d'obtenir des colonies *d'Haemophilus influenzae de type b* ayant une taille suffisante au bout de 16- 24 heures de culture (environ 3 à 5 mm).

Dans un des modes de réalisation préférée du procédé de production de PRP selon l'invention, le milieu solide comprend :
- au moins 1 mg/l de β NAD,
- au moins 0,5 mg/l de protoporphyrine IX,
- au moins une peptone végétale et un extrait de levure en quantité suffisante pour que la concentration en azote protéique dans le milieu solide soit d'au moins 0,2g/L et dans une proportion telle que le ratio entre la quantité de peptone végétale et la quantité d'extrait de levure dans le milieu soit de 0,1 à 9 lorsque la concentration en azote protéique du milieu est de 0,2 g/l à 0,8 g/l et qu'il soit de 1 à 9 lorsque la concentration en azote protéique du milieu est > 0,8 g/l,
- un carbohydrate,
- un agent détoxifiant et,
- un cocktail d'ions minéraux comprenant les ions Na⁺, K ⁺, Ca⁺⁺, Mg⁺⁺, Fe⁺⁺⁺, HPO₄⁻⁻, H₂PO4⁻, SO₄^{- -}, et Cl⁻ sous forme de solutions salines de sorte que le pH du milieu de culture se situe entre 6,5 et 7,5, de façon préférée entre 7,0 et 7,5.

Le carbohydrate utilisé est de préférence un sucre d'origine non animale, métabolisable par la bactérie, tel que le fructose, le ribose, le xylose, le fucose, le glycerol, ou en particulier le glucose. On a obtenu de bons résultats avec du glucose à une concentration d'au moins 0,1 g/l. Habituellement, on utilise le glucose à une concentration de 0,1 g/l à 20 g/l et de façon préférée de 0,1 g/l à 10 g/l.

Les agents détoxifiants favorisent la croissance des bactéries en neutralisant les substances inhibitrices qui peuvent être présentes dans les préparations d'agar comme cela a été rapporté par Evans N. M et coll (J. Med. Microbiol. Vol 7, pp 305-309, 1974). Comme agent détoxifiant, on utilise de préférence, le charbon, l'amidon, le tween ^{®}, le polyvinyl alcool, l'oléate de sodium ou du dithionite. De bons résultats ont été observés avec du tween 80^{®} (monooleate de polyoxyethylene sorbitane) utilisé à une concentration de 0,5 à 10 mg/l alors qu'une composition de milieu solide sans tween® produit des colonies de petite taille indistinguables sur le plan morphologique. On observe également des colonies de petite taille indistinguables sur le plan morphologique lorsque la concentration en β NAD est inférieure à 1 mg/l, lorsque la concentration en protoporphyrine IX est inférieure à 0,5 mg/l ou lorsque la concentration en glucose est inférieure à 0,1 mg/l.

Pour assurer une bonne croissance et une bonne viabilité des colonies les quantités en extrait de levure et en peptone végétale sont telles que la concentration en azote protéique totale est d'au moins 0,2 g/l. Le ratio entre la quantité de peptone végétale et la quantité d'extrait de levure dans le milieu peut varier dans une large mesure allant de 0,1 à 9 tant que la concentration en azote protéique dans le milieu de culture n'excède pas 0,8 g/l. Par contre, à une concentration supérieure, il y a une mauvaise individualisation des colonies due à un mauvais étalement de la suspension bactérienne sur le milieu solide lorsque ce ratio est inférieur à 1.

En ensemençant ce milieu solide à base d'agar, avec une population hétérogène de bactéries *Haemophilus influenzae serotype b,* (c.a.d. qui contient à la fois des bactéries capsulées et non capsulées) on observe au bout de 18 à 24 heures de culture des colonies blanches et des colonies grises que l'on distingue par transparence à l'aide d'un faisceau de lumière blanche. Les colonies blanches produisent plus de PRP que les colonies grises. Par ailleurs, les colonies blanches produisent également plus de PRP que les colonies provenant d'un milieu solide à base de charbon agar complémenté en sang de cheval (cf. exemple 2). Cette composition de milieu est considérée comme une composition de milieu solide sélective puisqu'elle permet le tri des colonies qui produisent le plus de PRP.

Selon un mode de réalisation encore plus préféré du procédé selon l'invention, le milieu solide comprend:
De 5 à 50 mg/l de β NAD,
De 0,5 à 5 mg/l de protoporphyrine IX,
De 1 à 10 g/l de glucose,
De 1 à 10 mg/l de Tween 80,
De 3 à 4 g/l de K₂HPO₄,
De 0,9 à 3 g/l de KH₂PO₄,
De 0,5 à 2 g/l de K₂SO₄,
De 20 à 500 mg/l de MgCl₂,
De 2 à 50 mg/l de CaCl₂, 2H₂O,
De 1 à 5 mg/l de FeCl₃, 6H₂O,
De 4 à 8 g/l de NaCl,
De 4 à 8 g/l d'un extrait de levure, et
De 4 à 8 g/l d'une peptone de petit pois de telle sorte que le ratio entre la quantité de la peptone de petit pois et la quantité d'extrait de levure soit ≥ 1 lorsque la concentration en azote protéique du milieu est > 0,8 g/l.

Les colonies blanches obtenues à partir de cette composition de milieu produisent jusqu'à 400 fois plus de PRP que les colonies grises. On a étudié leur locus *cap* en digérant l'ADN génomique à l'aide des enzymes de restriction Smal et KpnI. On a ensuite réalisé sur le produit de digestion une électrophorèse en champ pulsé suivi d'une révélation à l'aide d'une sonde spécifique PvuII selon les conditions opératoires décrites dans l'exemple 3. De façon surprenante, tous les profils électrophorétiques des colonies blanches comportent une bande électrophorétique de 45 Kb. On n'observe pas de bande électrophorétique de 18 Kb. Le locus *cap* de ces colonies contient par conséquent au moins deux copies du gène de 18 Kb ce qui signifie que la population bactérienne issue des colonies blanches est totalement capsulée. Par contre, les profils électrophorétiques des colonies grises comportent principalement une bande électrophorétique de 18 Kb. Cette composition de milieu sélective particulièrement préférée permet en outre de sélectionner des colonies blanches dont les populations bactériennes sont entièrement capsulées.

En définitive, un des moyens supplémentaires pour augmenter les rendements dans le procédé de production de PRP qui comporte une phase préliminaire de culture en milieu solide, consiste à transférer dans un milieu liquide uniquement les colonies blanches qui ont été obtenues à partir d'une composition de milieu solide sélective. De façon préférée, on utilise une composition de milieu solide qui permet d'obtenir des colonies blanches constituées essentiellement de bactéries capsulées.

C'est pourquoi l'invention a également pour objet, dans un mode de réalisation préféré, un procédé de production de PRP dans lequel on transfère dans le milieu de culture liquide uniquement les colonies blanches obtenues sur une composition de milieu solide sélective.

Dans un mode de réalisation particulièrement préféré, on transfère ces colonies blanches dans un milieu de culture liquide qui exerce un rôle stabilisant sur la population bactérienne capsulée. Outre le fait que toutes les étapes de culture sont effectuées avec des milieux dans lesquels la source d'azote protéique est d'origine non animale, ou même avec des milieux exempts de tout contaminant d'origine animale lorsqu'on utilise notamment de la protoporphyrine IX synthétique, ce procédé permet également d'optimiser la production de PRP lorsque la population d*'Haemophilus influeuzae de sérotype b* est hétérogène et contient à la fois des bactéries capsulées et des bactéries non capsulées. L'étape de culture sur milieu solide permet de sélectionner les colonies blanches qui renferment une population de bactéries entièrement capsulées. L'étape d'amplification de la biomasse dans le milieu liquide stabilise, comme on l'a vu précédemment, la population de bactéries capsulées en empêchant le développement de révertants non capsulés. Les rendements en PRP/ litre de culture finalement obtenus sont alors maximaux (voir exemple 4).

Ce procédé peut servir aussi à la production d'une population de bactéries entièrement capsulées. La population bactérienne obtenue est entièrement capsulée lorsque le profil électrophorétique de l'ADN génomique de cette population montre que le locus *cap* contient au moins deux copies du gène de 18 Kb (cf protocole de l'exemple 3) et que l'ensemencement d'une aliquote de cette population sur une composition de milieu solide sélectif selon l'invention produit plus de 95% de colonies blanches, et de façon préférée au moins 98% de colonies blanches. Après amplification des bactéries dans un milieu liquide stabilisant (c.a.d. qui empêche l'apparition de bactéries révertantes non capsulées), la population bactérienne obtenue est conservée par lyophilisation ou par congélation (dans ce cas on ajoute au milieu de culture un agent de congélation d'origine non animale tel que le glycérol). On réalise ainsi des lots de semence renfermant une population homogène de bactéries entièrement capsulées et qui offrent une garantie supplémentaire de sécurité biologique puisqu'ils ont été obtenus en utilisant des milieux de culture exempts de tout contaminant d'origine animale. Ces lots de semence peuvent servir à leur tour pour produire du PRP.

L'invention a donc pour objet :
- Un procédé de production de PRP, dans lequel toutes les étapes sont réalisées au moyen de milieux dépourvus de tout contaminant d'origine animale.
- Un procédé de production d'une population de bactéries entièrement capsulées d'*Haemophilus influenzae de sénotype b* dans lequel :
   (i) on cultive *Haemophilus influenzae de sérotype b* sur un milieu solide comprenant :
      - de 5 à 50 mg/l de β NAD,
      - de 0,5 à 5 mg/l de protoporphyrine IX,
      - de 1 à 10 g/l de glucose,
      - de 1 à 10 mg/l de Tween 80,
      - de 3 à 4 g/l de K₂HPO₄,
      - de 0,9 à 3 g/l de KH₂PO₄,
      - de 0,5 à 2 g/l de K₂SO₄,
      - de 20 à 500 mg/l de MgCl₂,
      - de 2 à 50 mg/l de CaCl₂, 2H₂O,
      - de 1 à 5 mg/l de FeCl₃, 6H₂O,
      - de 4 à 8 g/l de NaCl,
      - de 4 à 8 g/l d'un extrait de levure et,
      - de 4 à 8 g/l d'une peptone de petit pois de telle sorte que le ratio entre la quantité de peptone de petit pois et la quantité d'extrait de levure soit ≥ 1 lorsque la concentration en azote protéique du milieu est > 0,8 g/l ;
   (ii) on transfère et on cultive une ou plusieurs colonies blanches obtenues en (i) dans un milieu de culture liquide comprenant :
      - de 0,1 mg/l à 5 mg/l de protoporphyrine IX,
      - de 2 à 50 mg/l de β NAD,
      - de 2 à 20 g/l de glucose,
      - de 2 à 5 g/l d'un extrait de levure,
      - une peptone de petit pois équivalent à une concentration en azote protéique de 0,4 g/l à 1,5 g/l, et
      - un cocktail d'ions minéraux : Na⁺, NH4 ⁺, Ca⁺⁺, Mg⁺⁺, HPO₄⁻⁻, H₂PO₄-, SO₄⁻⁻, et Cl⁻ sous forme de solutions salines de sorte que le pH du milieu se situe entre 6,5 et 7,5, de façon préférée entre 7,0 et 7,5 et,
   (iii) on congèle ou on lyophilise la culture bactérienne obtenue en (ii).
      Enfin
      - Un procédé de production d'une population de bactéries entièrement capsulées d'*Haemophilus influenzae de sérotype b,* dans lequel toutes les étapes sont réalisées au moyen de milieux dépourvus de tout contaminant d'origine animale.

Elle a également pour objet l'utilisation d'une population homogène de bactéries capsulées d'*Haemophilus influenzae de sérotype b* obtenues selon ce procédé, pour la production de PRP.

L'invention a pour objet un vaccin contre la méningite à *Haemophilus influenzae de type b* comprenant du PRP obtenu à partir d'un des modes de réalisation du procédé selon l'invention.

L'invention a finalement pour objet un milieu de culture solide *d'Haemophilus influenzae serotype b,* dont la source d'azote protéique est d'origine non animale et qui comprend:
- au moins 1 mg/l de β NAD,
- au moins 0,5 mg/l de protoporphyrine IX,
- une peptone et un extrait de levure en quantité suffisante pour que la concentration en azote protéique dans le milieu soit d'au moins 0,2g/l d'azote protéique et dans une proportion telle que le ratio entre la quantité de peptone végétale et la quantité d'extrait de levure dans le milieu soit de 0,1 à 9 lorsque la concentration en azote protéique du milieu est de 0,2 g/l à 0,8 g/l et qu'il soit de 1 à 9 lorsque la concentration en azote protéique du milieu est > 0,8 g/l,
- un carbohydrate,
- un agent détoxifiant, et
- un cocktail d'ions minéraux : Na⁺, K ⁺, Ca⁺⁺, Mg⁺⁺, Fe⁺⁺⁺, HPO₄⁻, H₂PO4⁻, SO₄^{- -}, et Cl⁻ sous forme de solutions salines de sorte que le pH du milieu se situe entre 6,5 et 7,5, de façon préférée entre 7,0 et 7,5.

De façon préférée le milieu de culture solide comprend:
- de 5 à 50 mg/l de β NAD,
- de 0,5 à 5 mg/l de protoporphyrine IX,
- de 1 à 10 g/l de glucose,
- de 1 à 10 mg/l de Tween 80,
- de 3 à 4 g/l de K₂HPO₄,
- de 0,9 à 3 g/l de KH₂PO₄,
- de 0,5 à 2 g/l de K₂SO₄,
- de 20 à 500 mg/l de MgCl₂,
- de 2 à 50 mg/l de CaCl₂, 2H₂O,
- de 1 à 5 mg/l de FeCl₃, 6H₂O,
- de 4 à 8 g/l de NaCl,
- de 4 à 8 g/l d'un extrait de levure et,
- de 4 à 8 g/l d'une peptone de petit pois de telle sorte que le ratio entre la quantité de peptone de petit pois et la quantité d'extrait de levure soit ≥ 1 lorsque la concentration en azote protéique du milieu est > 0,8 g/l.

La présente invention sera mieux comprise à la lumière des exemples suivants qui servent à illustrer l'invention sans pour autant en limiter le contenu.

La figure 1 représente les taux de production en PRP (en mg/l) dans un milieu de culture dans lequel les peptones d'origine animale ont été remplacées par de la peptone de pois en fonction des concentrations en hémine (-◆-) ou en protoporphyrine IX d'origine animale (-■-) ou synthétique (-▲-) (en µg/L).

La figure 2 représente les taux de production en PRP (en mg/L) dans un milieu de culture dans lequel les peptones d'origine animale ont été remplacées par de la peptone de blé en fonction des concentrations en hémine (-◆-) ou en protoporphyrine IX d'origine animale (-■-) ou synthétique (-▲-) en µg/L).

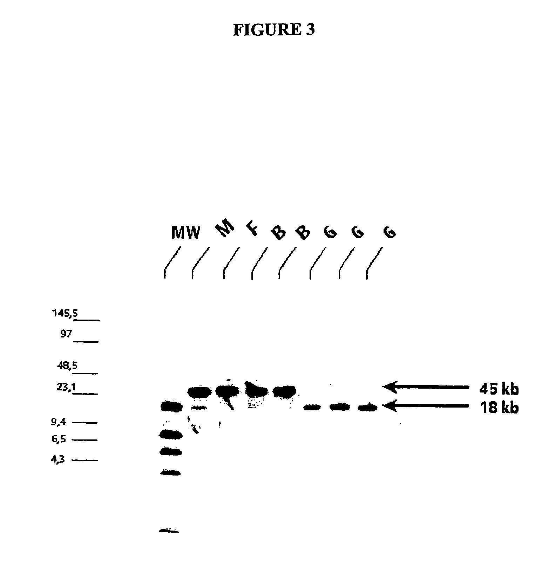

La figure 3 représente les profils électrophorétiques de différentes populations bactériennes *d'Haemophilus influenzae sérotype b* : la bande M représente le profil électrophorétique d'une population mère hétérogène avec deux bandes de 18 Kb et 45 Kb, la bande F le profil électrophorétique de sa population fille (F) après sélection sur milieu solide sélectif avec une seule bande de 45 Kb ; les bandes B représentent les profils de différentes colonies bactériennes blanches (B) avec une seule bande de 45 Kb et les bandes G, les profils de différentes colonies bactériennes grises (G) avec une seule bande de 18 Kb.
La bande MW montre le positionnement des marqueurs de poids moléculaires (Kb).

### Exemple 1 : influence de la protoporphyrine IX sur la production de PRP dans un milieu de culture à base de peptone végétale.

### 1) Méthodologie

On a comparé la production de PRP obtenue au bout de 16 heures de culture bactérienne dans des milieux de culture liquides à base de peptones végétales dans lesquels la source d'hème est soit de l'hémine, soit de la protoporphyrine IX d'origine animale (protoporphyrine porcine) sous forme de sel disodique, soit de la protoporphyrine IX d'origine purement synthétique sous forme de sel disodique. Les gammes de concentration en hémine et protoporphyrine IX dans les milieux de culture varient d'environ 0,010 g/L à environ 2 g/L de façon à obtenir des courbes de titrage en PRP en fonction de la source d'hème testée et en fonction de la peptone végétale testée. On a testé un hydrolysat enzymatique de peptone de petit pois fournie par Kerry (Hy pea 7404) et une peptone de blé fournie par Organotechnie (19559) à une concentration dans le milieu de culture équivalent à 0,87 g/l d'azote protéique. Par référence aux conditions actuelles de production de PRP, on a également mesuré la production de PRP dans un milieu qui contient une peptone d'origine animale, telle que l'hydrolysat de caséine (HAC) fourni par Solabia à une concentration équivalente à 0,87g/l d'azote protéique en présence de concentrations croissantes en hémine (cf tableau 3).

### 1.1) Préparation des milieux

1.1.1 Solution mère de β NAD (Fluka) à 1g/L en eau ultrafiltrée puis stérilisée par filtration sous 0,22 µm.
1.1.2 Solution mère d'hémine (Sigma) à 0,25 g/L en eau ultrafiltrée comprenant 5ml d'ammoniaque à 25% (Cooper) pour faciliter la dissolution. La solution mère est stérilisée par filtration 0,22µm.
1.1.3 Solution mère de protoporphyrine IX porcine (Sigma) à 0,25 g/L en eau ultrafiltrée comprenant 5ml d'ammoniaque à 25% (Cooper) pour faciliter la dissolution. La solution mère est stérilisée par filtration 0,22µm.
1.1.4 Solution mère de protoporphyrine IX synthétique à 0,25 g/L en eau ultrafiltrée comprenant 5ml d'ammoniaque à 25% (Cooper) pour faciliter la dissolution. La solution mère a également été chauffée à 80°C dans un bain marie sous agitation pour compléter la dissolution avant d'être stérilisée par filtration 0,22µm. La protoporphyrine IX, sous forme de sel disodique, a été synthétisée selon la méthode décrit dans la demande de brevet française enregistrée sous le n° d'enregistrement 07/02334 et déposé le 30/03/2007 en utilisant les étapes qui sont décrites dans le schéma 2.

Les solutions mères d'hémine et de protoporphyrine IX ont été contrôlées pour leurs teneurs respectives en composés actifs après filtration stérilisante. Les teneurs en protoporphyrine IX et en hémine ont été déterminées par Chromatographie Liquide Haute Performance en phases inversées (RP-HPLC). La chaîne chromatographique comprend un module de pompe à deux têtes permettant la réalisation d'un gradient binaire, un injecteur automatique programmable, un détecteur UV à barrette de diodes et une colonne chromatographique de type Synergi 4µm, Polar RP-80A (150x4,6) mm, ref 00F-4336-E0, Phenomenex.

La Solution mère d'hémine (Sigma) à contrôler après filtration est diluée au 1/3 dans l'eau distillée. Les solutions mères de protoporphyrine IX porcine (Sigma) et synthétique à contrôler après filtration sont diluées au 1/4 dans l'eau distillée. En parallèle, on réalise une gamme d'étalonnage en eau ammoniaquée allant de 0,025 g/L à 0,125 g/L de protoporphyrine IX à partir de la protoporphyrine IX porcine de Sigma (ref: 25838-5) et une gamme d'étalonnage allant de 0,050 g/L à 0,150 g/L d'hémine à partir de l'hémine de Sigma (ref: H5533-256). Les échantillons à contrôler ainsi que les différentes solutions de la gamme d'étalonnage sont injectés sous un volume de 20µl (pour les solutions et échantillons d'hémine) ou de 5µl (pour les solutions et échantillons de protoporphyrine IX). La phase mobile initiale constituée d'un mélange volume à volume d'Acétonitrile et de KH2PO4 10mM pH2,5 est réglée à un débit de 1ml/min. On réalise ensuite un gradient discontinu à partir de cette phase mobile pour séparer les molécules d'intérêt qui sont détectées à une longueur d'onde de 400nm. Après avoir établi la gamme d'étalonnage et sur la base de la surface des pics des échantillons à contrôler on en a déduit la concentration en hémine et en protoprphyrine IX dans les différentes solutions mères filtrées qui étaient respectivement de 0,295 g/l pour la solution d'hémine, de 0,187 g/l pour la solution de protoporphyrine IX porcine et de 0,249 g/l pour la solution de protoporphyrine IX synthétique. Ces concentrations n'ont pas été ajustées par la suite à la concentration cible de 0,250g/l mais on s'est basé sur ces concentrations réellement présentes dans les solutions mère d'hémine et - protoporphyrine IX dans l'analyse des résultats.
1.1.5 Solution mère d'extrait autolytique de levure (Solabia) à 125 g/L en eau ultrafiltrée puis stérilisée sous 0,22 µm.
1.1.6 Solution mère de glucose à 465,12g/L en eau ultrafiltrée puis stérilisée par filtration sous 0,22 µm.
1.1.7 Solution d'enrichissement

Elle est constituée de 40 ml de solution mère d'extrait de levure, de 43 ml de solution mère de glucose et de 5 ml de solution mère de β NAD.

1.1.8 Milieu de base
- peptone végétale de blé (Organotechnie-Ref 19559) ou peptone végétale de petit pois (Kerry-Ref Hypea 7404) en quantité suffisante pour apporter l'équivalent de 0,95 g d'azote protéique par litre de milieu de base, la quantité d'azote protéique étant dosé selon la méthode kjedhal,
- lactate de sodium en solution aqueuse à 50% (VWR): 1,8 ml,
- hydrogenophosphate disodique , 12H₂O (Budenheim): 31,14 g,
- dihydrogenophosphate de sodium, 2H₂O (Merck: 2,03 g ,
- L cystine (Jera France): 0,07 g,
- HCl à 37% (VWR): 0,07 ml,
- L tryptophane (Jera France): 0,02 g,
- sulfate d'ammonium: 1 g,
- sulfate de magnesium, 7H₂O: 0,4g,
- chlorure de calcium, 2H₂O : 0,02 g
- eau ultra filtrée : quantité suffisante pour 1 litre.

Le milieu de base est finalement stérilisé par autoclavage à 121°C pendant 30 minutes.

### 1.2) Protocole opératoire

La culture a été réalisée en Erlens de 500 mL. Dans chaque Erlen, on introduit 100 ml de milieu de base contenant soit de la peptone de blé, soit de la peptone de petit pois, 8,8 ml de milieu d'enrichissement et un volume variable de la solution mère d'hémine, de protoporphyrine IX porcine ou de protoporpyrine IX synthétique de façon à ce que les concentrations théoriques en hémine (ou en protoporphyrine IX) testées dans les différents milieux de culture se situent entre 10µg/L et environ 2000µg/L (voir tableaux I et II). On inocule chaque Erlen avec le contenu d'un congelât *d'Haemophilus influenzae* de serotype b qui contient 10⁸ à 10¹⁰ bactéries/ml à un taux d'inoculation de 0,2% (V/V) Au bout de 16 heures d'incubation sous agitation à 175 rpm dans un incubateur à 37°C on mesure, dans chaque Erlen, la D.O. de la suspension bactérienne obtenue et la concentration en PRP en prélevant un peu de surnageant de culture.

### 1.3) Dosage du PRP

La productivité en PRP a été déterminée sur la base d'un dosage ELISA de type sandwich effectué en double sur les surnageants de culture.

Les microplaques ELISA sont sensibilisées pendant une nuit à +4°C en introduisant dans chaque puits 100µL d'une solution d'immunsérums de lapins hyperimmunisés avec des germes d'*Haemophilus influenzae* de type b, préalablement diluée dans un tampon carbonate 0,2M pH 9,6 (dilution =1/2000). Après rinçage et saturation des microplaques ELISA, on réalise dans chaque microplaque une gamme d'étalonnage à partir d'une solution purifiée de PRP à 1mg/ml en eau distillée en réalisant des dilutions successives dans un tampon de dilution (PBS/Tween 20 0,05%/sérum albumine bovine 1%). On introduit également les surnageants de culture à doser en réalisant également des dilutions successives dans le tampon de dilution. Après une nouvelle incubation d'environ 2 heures à 37°C, suivie d'une phase de rinçage des microplaques, on introduit dans les micropuits 100µL d'une solution d'anticorps de lapins biotinylés obtenus en traitant les sérums de lapins vaccinés avec le vaccin *Haemophilus Influenzae type b* conjugué à la protéine tétanique avec un agent de biotinylation, préalablement diluée dans le tampon de dilution (dilution =1/500). Après une incubation pendant 1 heure à 37°C suivie d'une étape de rinçage on rajoute dans chacun des micropuits 100µl d'une solution de streptavidine couplée à la peroxydase (Southern Biotechnogy- ref 7100-05) préalablement diluée dans le tampon de dilution (dilution ≈1/5000). Après une incubation pendant 1 heure à 37°C suivie d'une étape de rinçage on rajoute dans chaque micropuits 100µl d'une solution de révélation (solution d'orthophénylène diamine à 0,4mg/ml en tampon phosphate-citrate 0,05M, pH=5 additionnée de 0,3µl de peroxyde d'hydrogène à 0,03%). Après un temps de révélation de 20 minutes à l'abri de la lumière on bloque la réaction en rajoutant 50µl/puits d'H2SO4 2N. La lecture des microplaques se fait à 492 et 620nm (pour tenir compte de l'absorption du plastique). A partir des densités optiques obtenues sur les échantillons testés, on détermine par interpolation au moyen de la gamme d'étalonnage, la teneur en PRP dans les différents surnageants de culture.

### 1.4) Résultats

Les résultats sont représentés dans les tableaux let 2 ci-dessous ainsi que dans les figures 1 et 2.

**Tableau 1 :**

| **protoporphyrine IX porcine en µg/L** | **Pois + protoporphyrine IX porcine** | | | |
|---|---|---|---|---|
| | **PRP en mg/L Dosage 1** | **PRP en mg/L Dosage 2** | **Moyenne PRP en mg/L** | **Ecart type PRP** |
| **7,48** | 15,1 | 16,1 | **15,6** | 0,71 |
| **37,4** | 143 | 149 | **146** | 4,24 |
| **74,8** | 344 | 354 | **349** | 7,07 |
| **187** | 450 | 490 | **470** | 28,28 |
| **374** | 415 | 432 | **423,5** | 12,02 |
| **748** | 495 | 523 | **509** | 19,80 |
| **1122** | 492 | 550 | **521** | 41,01 |
| **1496** | 466 | 493 | **479** | 19,09 |

| **protoporphyrine IX synthétique en µg/L** | **Pois + Protoporphyrine IX synthétique** | | | |
|---|---|---|---|---|
| | **PRP en mg/L Dosage 1** | **PRP en mg/L Dosage 2** | **Moyenne PRP en mg/L** | **Ecart type PRP** |
| **9,96** | 11,3 | 11,8 | **11,55** | 0,35 |
| **49,8** | 139 | 134 | **136,5** | 3,54 |
| **99,6** | 330 | 383 | **356,5** | 37,48 |
| **249** | 474 | 502 | **488** | 19,80 |
| **498** | 383 | 392 | **387,5** | 6,36 |
| **996** | 524 | 474 | **499** | 35,36 |
| **1494** | 503 | 523 | **513** | 14,14 |
| **1992** | 480 | 497 | **488,5** | 12,02 |

| **Hémine en µg/L** | **Pois + Hémine** | | | |
|---|---|---|---|---|
| | **PRP en mg/L Dosage 1** | **YRP en mg/L Dosage 2** | **Moyenne PRP en mg/L** | **Ecart type PRP** |
| **11,8** | 3,82 | 3,79 | **4** | 0,02 |
| **59** | 7,61 | 7,13 | **7** | 0,34 |
| **118** | 23,4 | 17,5 | **20** | 4,17 |
| **295** | 51,2 | 45,4 | **48** | 4,10 |
| **590** | 95,4 | 72,9 | 84 | 15,91 |
| **1180** | 234 | 232 | **233** | 1,41 |
| **1770** | 209 | 211 | **210** | 1,41 |
| **2360** | 505 | 482 | **494** | 16,26 |
| **3540** | 129 | 154 | **142** | 17,68 |

**Tableau 2 :**

| **protoporphyrine IX porcine en µg/L** | **Blé + protoporphyrine IX porcine** | | | |
|---|---|---|---|---|
| | **PRP en mg/L Dosage 1** | **PRP en mg/L Dosage 2** | **Moyenne PRP en mg/L** | **Ecart type PRP** |
| 7,48 | 25,5 | 28,3 | 26,9 | 1,98 |
| 37,4 | 136 | 149 | 142,5 | 9,19 |
| 74,8 | 429 | 299 | 364 | 91,92 |
| **187** | 452 | 408 | **430** | 31,11 |
| **374** | 397 | 374 | **385,5** | 16,26 |
| **748** | 399 | 377 | **388** | 15,56 |
| **1122** | 446 | 329 | **387,5** | 82,73 |
| **1496** | 464 | 283 | **373,5** | 127,9 |

| **protoporphyrine IX synthétique en µg/L** | **Blé + Protoporphyrine IX synthétique** | | | |
|---|---|---|---|---|
| | **PRP en mg/L Dosage 1** | **PRP en mg/L Dosage 2** | **Moyenne PRP en mg/L** | **Ecart type PRP** |
| **9,96** | 33,5 | 27,9 | **30,7** | 3,96 |
| **49,8** | 317 | 319 | **318** | 1,41 |
| **99,6** | 389 | 437 | **413** | 33,94 |
| **249** | 345 | 350 | **347,5** | 3,54 |
| **498** | 380 | 409 | **394,5** | 20,51 |
| **996** | 401 | 404 | **402,5** | 2,12 |
| **1494** | 358 | 350 | **354** | 5,66 |
| **1992** | 378 | 430 | **404** | 36,77 |

| **Hémine en µg/L** | **Blé + Hémine** | | | |
|---|---|---|---|---|
| | **PRP en mg/L Dosage 1** | **PRP en mg/L Dosage 2** | **Moyenne PRP en mg/L** | **Ecart type PRP** |
| 11,8 | 4,98 | 5,61 | 5,3 | 0,45 |
| 59 | 13 | 13,7 | 13,35 | 0,49 |
| 118 | 17,7 | 17,5 | 17,60 | 0,14 |
| **295** | 35,3 | 30,2 | **32,75** | 3,61 |
| **590** | 71,4 | 73,4 | **72,4** | 1,41 |
| **1180** | 327 | 256 | **291,5** | 50,2 |
| **1770** | 487 | 446 | **466,5** | 28,99 |
| **2360** | 487 | 504 | **495,5** | 12,02 |

**Tableau 3**

| **Hémine en µg/L** | **HAC+ Hémine** | | | |
|---|---|---|---|---|
| | **PRP en mg/L Dosage 1** | **PRP en mg/L Dosage 2** | **Moyenne PRP en mg/L** | **Ecart type PRP** |
| 11,8 | 18,9 | 14,7 | 16,8 | 2,97 |
| 59 | 82,7 | 77,7 | 80,2 | 3,54 |
| 118 | 183 | 216 | 199,5 | 23,33 |
| **295** | 325 | 304 | **314,5** | 14,85 |
| **590** | 381 | 440 | **410,5** | 41,72 |
| **1180** | 424 | 405 | **414,5** | 13,44 |
| **1770** | 404 | 519 | **461,5** | 81,32 |
| **2360** | 439 | 490 | **464,5** | 36,06 |

La figure 1 reproduit les résultats du tableau I en montrant les courbes de production en PRP obtenues en fonction de la concentration et de la source d'hème utilisée dans un milieu qui renferme de la peptone de petit pois. Les courbes de production en PRP sont équivalentes selon qu'on utilise comme source d'hème une protoporphyrine IX synthétique ou une protoporphyrine IX d'origine animale. Par contre la production en PRP est nettement plus basse lorsque le milieu renferme de l'hémine par comparaison avec un milieu qui renferme de la protoporphyrine IX et ceci dans toute la gamme de concentration testée. Il suffit de mettre environ 200µg/L de protoporphyrine IX pour avoir une production optimale en PRP (= 480mg/ml) tandis qu'il faut environ 2500µg/L d'hémine pour avoir une production maximale en PRP. Il faut donc environ 12,5 fois moins de protoporphyrine IX que d'hémine dans un milieu de culture à base de peptone de pois pour avoir une production maximale en PRP.

La figure 2 reproduit les résultats du tableau II en montrant les courbes de production en PRP obtenues en fonction de la concentration et de la source d'hème utilisée dans un milieu qui renferme de la peptone de blé. Les courbes de production en PRP sont équivalentes selon qu'on utilise comme source d'hème une protoporphyrine IX synthétique ou une protoporphyrine IX d'origine animale. Par contre la production en PRP est plus faible lorsque le milieu renferme de l'hémine par comparaison avec un milieu qui contient de la protoporphyrine IX à concentrations équivalentes et ceci apparait d'autant plus nettement que la concentration en hémine est faible. Il suffit de mettre environ 100µg/L de protoporphyrine IX pour avoir une production optimale en PRP (≈ 400mg/ml) tandis qu'il faut environ 1500µg/L d'hémine pour avoir une production équivalente en PRP. Il faut donc environ 15 fois moins de protoporphyrine IX que d'hémine dans un milieu de culture à base de peptone de blé pour obtenir les mêmes productivités en PRP.

Les résultats des tableaux I, II et III montrent également qu'il faut que la concentration en hémine dans un milieu à base de peptone animale et d'hémine, qui est pourtant la composition de milieu préconisée pour produire du PRP, soit 2 à 5 fois plus importante que la concentration en protoporphyrine IX nécessaire dans un milieu à base de peptone végétale telle que la peptone de blé ou de petit pois pour obtenir la même concentration en PRP dans le surnageant de culture. Par exemple, pour obtenir une concentration d'environ 400 mg/l de PRP dans un milieu à base d'hydrolysat de caséine et d'hémine il faut que la concentration en hémine soit d'au moins 500µg/l, alors qu'une concentration d'environ 100µg/l en protoporphyrine IX suffit dans un milieu à base de peptone de blé ou de petit pois et de protoporphyrine IX. Les milieux à base de peptone végétale et de protoporphyrine IX apparaissent donc plus avantageux que les milieux actuels renfermant une peptone animale et de l'hémine servant à la production de PRP.

### Exemple 2 : Influence de la composition du milieu solide sur la production de PRP par les colonies

On reprend un lyophilisat d'une population hétérogène de bactéries *Haemophilus influenzae serotype b* contenant environ 10⁸ germes dans 1ml de tampon PBS Dubelcco (Gibco ref 14040-083). On réalise des dilutions successives de raison 10 dans ce tampon. On prélève 50µl de chacune des dilutions: 10⁻⁵, 10⁻⁶ et 10⁻⁷ que l'on ensemence, soit sur des boîtes de pétri renfermant différentes compositions de milieux solides sélectifs selon l'invention, soit sur une boîte de pétri renfermant un milieu solide standard contenant une gélose charbon agar (Difco, Réf 289410) additionnée de 10% (V/V) de sang de cheval cuit défibriné (BioMérieux, Réf 55832).

Après une incubation d'une nuit à 37°C dans une étuve contenant 10% de CO₂, on examine les colonies par transparence, sous une lampe de 75 W, les boîtes de Pétri étant fermées. Les colonies apparaissent opaques et uniformes sur le milieu standard. Par contre, on observe des colonies blanches et des colonies grises sur les milieux sélectifs. On a prélevé au hasard 4 colonies du milieu solide standard ainsi que 4 colonies grises et 4 colonies blanches sur deux des milieux sélectifs testés (A et B) dont les compositions par litre sont les suivantes :

| | Milieu sélectif A | Milieu sélectif B |
|---|---|---|
| β NAD: | 10 mg | 10 mg |
| protoporphyrine IX : | 5 mg | 0,5 mg |
| glucose : | 1 g | 1 g |
| Tween 80 : | 1 mg | 1 mg |
| K₂HPO₄ : | 3g | 3g |
| KH₂PO₄ : | 0,94 g | 0,94 g |
| K₂SO₄ | 0,5 g | 0,5 g |
| MgCl₂ : | 0,5 g | 0,5 g |
| CaCl₂, 2H₂O: | 0,002 g | 0,002 g |
| FeCl₃, 6H₂O : | 0,005 g | 0,005 g |
| NaCl : | 8g | 8g |
| Extrait de levure (Difco-ref : 212 740) : | 5 g | 5 g |
| Peptone de petit pois sous forme d'hydrolysat acide (Oxoid) : | 5,3 g | 5,3 g |
| Bacto agar : | 15 g | 15 g |

Sur chaque colonie prélevée, on dose le contenu en PRP par chromatographie Chromatographie Haute Performance Couplée à une Détection Ampérométrique en champs pulsés (HPAEC/PAD)

La quantification du PRP se fait par dosage du ribitol qui est un des composants de l'unité répétitive du polysaccharide et qui est libéré quantitativement après hydrolyse acide.

### 2.1) Préparation des échantillons

Chaque colonie est reprise dans 500 µl d'eau ultrafiltrée puis on prélève 100 µl de la suspension que l'on dilue dans 300 µl d'eau ultrafiltrée.

### 2.2) Préparation de la gamme étalon

A partir d'une solution mère de ribitol à 1 mg/l en eau purifiée ultra filtrée, on réalise une gamme étalon en ribitol allant de 0 à 20 µg/ml. Le volume final de chaque échantillon de la gamme étalon est également de 400 µl.

### 2.3) Hydrolyse acide

On ajoute 100 µl d'une solution d'acide trifluoroacétique 10 N à chaque préparation d'échantillon ou à chaque échantillon de la gamme étalon. L'hydrolyse est effectuée pendant 2 heures à 120° C. On sèche ensuite tous les tubes sous un courant d'azote et on reprend chaque dessicat par 400 µl d'eau purifiée ultra filtrée au moment de l'analyse.

### 2.4) Analyse par chromatographie HPAEC-PAD

On injecte 100 µl de chacun des hydrolysats sur une colonne analytique CARBOPAC MA1 (4X250 mm) (DIONEX # 44066) préalablement équilibrée par une solution de soude 480 mM. On soumet la colonne à un flux d'une solution contenant 48% de soude 1 M et 52% d'une solution d'eau purifiée ultra filtrée pendant 40 minutes à un débit de 0,4 ml/min pour éluer les deux monosaccharides constitutifs du PRP. La température de la colonne est maintenue à 30°C pendant toute la durée de l'analyse. Les monosaccharides sont détectés à l'aide d'un détecteur électrochimique multimodes ED40 couplé à une cellule ampérométrique (DIONEX #44094).

Dans ces conditions le pic de chromatographie correspondant au ribitol libéré lors de l'hydrolyse du PRP apparaît à 19 ± 5% min.

On établit la courbe d'étalonnage (quantité de ribitol en fonction de la surface des pics chromatographiques) à partir de la gamme d'étalonnage puis on détermine par interpolation la quantité de ribitol contenue dans chacune des préparations d'échantillons. On en déduit la quantité de PRP que contient chaque échantillon puis la concentration de PRP dans chaque colonie sachant que le ribitol représente 41% du poids de PRP.

### 2.5) Détermination de la biomasse des colonies

Le contenu en protéines de chaque colonie, déterminé selon la méthode de MicroBCA (Pierce), reflète la biomasse de chaque colonie. Pour cela, les colonies sont prélevées individuellement puis reprises dans 200 µl d'eau ultrafiltrée stérile. Le mélange est agité au vortex 30 secondes. Des prises d'essais (10 µl à 40 µl) sont effectuées pour réaliser le dosage protéique à l'aide du kit MicroBCA (pierce) selon les recommandations du fabricant. Une gamme étalon est préparée à partir d'une solution de sérumalbumine bovine à 100 µg/ml. La lecture des échantillons et de la gamme étalon s'effectue au spectrophotomètre à 562 nm. Les concentrations protéiques des échantillons exprimées en µg/colonie sont calculées à l'aide de la gamme étalon.

### 2.6) Résultats

Les résultats exprimés en µg de PRP par unité de masse protéique (exprimée en µg) sont répertoriés dans le tableau ci dessous.

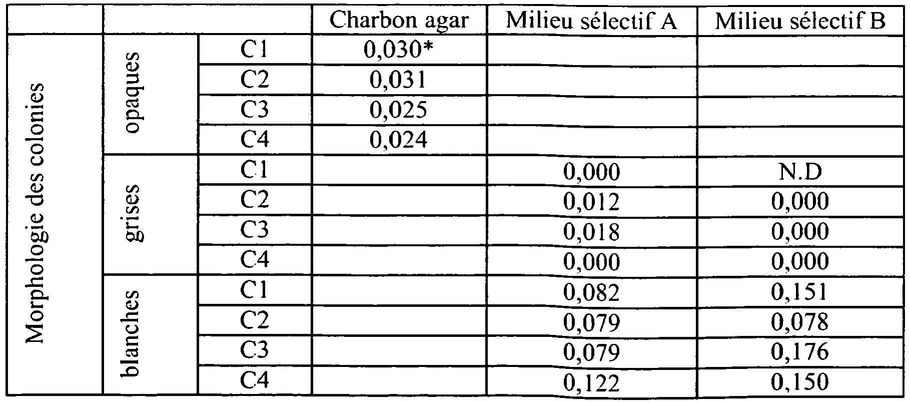

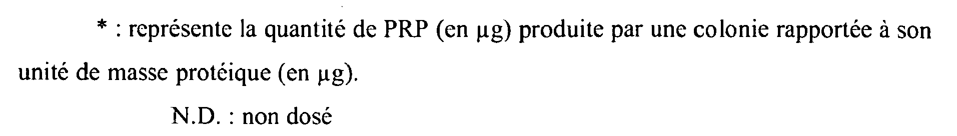

La production de PRP par les colonies est d'autant plus importante que la quantité de PRP dosée par unité de masse protéique est importante Ces résultats montrent que la production de PRP par les colonies blanches est 3 à 5 fois plus grande que la production des colonies prélevées sur charbon agar. Par contre, les colonies grises produisent généralement de plus faibles quantités de PRP que les colonies prélevées sur charbon agar.

### Exemple 3 : Influence de l'étape de culture sur milieu solide sélectif sur la production de PRP en milieu liquide

On a comparé deux procédés de production de PRP. Dans le premier procédé, on ensemence le contenu d'un congelât (≈10⁸ bactéries/ml) d'une population de bactéries *Haemophilus influenzae type b,* dénommée population mère directement dans un milieu de culture liquide selon l'invention. Les caractéristiques de la population mère ont été au préalable analysées (voir paragraphe suivant). Dans le second procédé, on dérive une population fille à partir de la population mère en utilisant un milieu solide sélectif selon l'invention qui permet de sélectionner une population fille à partir des colonies blanches qui contiennent 100% de bactéries capsulées. La population fille est ensuite ensemencée dans le même milieu de culture que la population mère. On a ensuite mesuré et comparé dans une troisième étape la production de PRP par la population mère et la population fille.

### 3.1) Caractéristiques de la population mère

### 3.1.1 : analyse du locus cap

### 3.1.1.1 : réactifs

### Tampons de lyse bactérienne

- Tampon Pett IV : 10 mM Tris-HCl pH 7,4, 1 M NaCl
- solution de lyse 1X : 6 mM Tris-HCL pH 7,4, 1 M NaCl, 10mM EDTA, 0,5% Brij 58, 0,2% Sarkosyl, 5mg/ml Lysosyme, 1µg/ml Rnase
- solution ESP : 10 mM Tris-HCL pH 7,4, 1mM EDTA, 1% SDS, 1 mg/ml protéinase
- solution TE : 10 mM Tris-HCl pH 7,4, 0,1 mM EDTA

### Digestion enzymatique

*-SmalI :* (GIBCO -BRL Réf: 15228-018)
tampon de digestion 4 10X : (GIBCO BRL, fourni avec l'enzyme)- à diluer 10 fois en eau purifiée stérile dépourvue de nuclease au moment de l'emploi
*-KpnI* : (INVITROGEN Réf: 155232-036)
Tampon de digestion 4 10X : (INVITROGEN Réf: 155232-036)- à diluer 10 fois en eau purifiée stérile dépourvue de nuclease au moment de l'emploi
Tampon d'électrophorèse en champ pulsé
tampon TBE 10X : 890 mM Tris-HCL pH 7,4, 890 mM Acide borique, 250 mM EDTA pH 8,0- à diluer 20 fois en eau ultra filtrée au moment de l'emploi

### Sonde PvuII marquée à la digoxygénine :

La sonde spécifique PvuII marquée a été obtenue à partir d'une préparation d'ADN provenant du plasmide pBR322-pU038 (département de pédiatrie -Université d'oxford- John Radcliffe Hospital). 20µg d'ADN plasmidique sont digérés pendant 2 heures à 37°C en présence de 40 unités d'enzyme *pvuII* (NEBIOLABS Réf. #R0151-S) dans un tampon 4 10X (NEBIOLABS Réf. #B7002-S) préalablement dilué 10 fois en eau stérile dépourvue de nuclease. Le produit de digestion a été ensuite soumis à une électophorèse sur gel d'agarose à 1% Poids/volume en présence d'un tampon TAE IX dans lequel on a ajouté 0,25% volume/volume bleu de bromophénol, 0,25% volume/volume de xylène cyanol FF, 30% volume/volume de glycérol. On prélève à la fin de la migration la bande d'intérêt de 2,1 Kb correspondant au fragment d'ADN PvuII. On extrait ensuite l'ADN du gel d'agarose par passage sur une colonne « Nucleospin » (Macherey -Nalgel Réf : 740590.250) puis on contrôle son intégrité par lecture spectrophotométrique à 260 nm. Finalement la sonde PvuII est marquée à la digoxygénine en utilisant le kit de marquage « DIG-Chem-Link Labeling and Detection Set » (ROCHE Réf : 1836463). On conserve la sonde marquée à -20°C.

### 3.1.1.2 : protocole opératoire

On décongèle une ampoule de la population mère que l'on ensemence sur une boîte de pétri renfermant un milieu solide standard constitué d'une gélose charbon agar (Difco, ref 289410) additionnée de 10% (V/V) de sang de cheval cuit défibriné (BioMérieux, ref 55832). Après une incubation de 18h à 37° dans une étuve contenant 10% de CO₂, on récolte les colonies obtenues que l'on suspend dans un tampon Tampon Pett IV de sorte que la D.O. 680 nm soit ≈ 1,8. On mélange la suspension bactérienne volume à volume avec de l'agarose à 2% (V/V) à bas point de fusion (Réf: BioRad, ref 162-0138), tempéré à 50°C puis on répartit ce mélange dans des moules de «plugs » (BioRad Réf: 170-3713) à raison de ≈ 80 µl/ « plug ». On obtient ainsi des moules d'agarose contenant le germe entier. Chaque « plug » est placé dans 1 ml de solution de lyse 1X. Après une incubation de 6h à 37°C on remplace cette solution par 1ml d'une solution ESP. Après une nouvelle incubation d'une nuit à 50°C, on lave 3 fois chaque « plug » avec 4 ml d'une solution TE pendant 30 min. L'ADN génomique des bactéries lysées contenu dans chaque plug est ensuite digéré pendant 1 nuit à 25°C à l'aide de 300µl d'un tampon de digestion 4 1X (GIBCO BRL) contenant 20 unités de l'enzyme *SmaI* (GIBCO -BRL Réf: 15228-018) puis lavé dans 4 ml d'une solution de TE. On poursuit la digestion pendant 7 heures à 30°C à l'aide de 300µl d'un tampon 4 IX (INVITROGEN Réf: 155232-036) contenant 20 unités de l'enzyme *KpnI* (INVITROGEN ref: 155232-036) suivi d'un lavage dans une solution de TE. Ces deux enzymes de restriction libèrent le locus *cap* de l'ADN génomique bactérien. Les « plugs » digérés sont insérés dans un gel d'agarose certifié à 0,8%V/V (BIORAD ref: 162-0138) puis soumis à une électrophorèse en champ pulsé réalisée en tampon TBE 0,5X pendant 13 heures, en utilisant un appareil de type « Chef mapper » (Biorad) réglé de sorte que soient appliqués 6 volt/cm, un angle de 120°, une progression linéaire, un temps de « switch » initial de 0,09s , et un temps de « switch final » de 11,54 secondes. Le gel est transféré sur filtre de nylon chargé positivement (Roche Réf: 1209272) par transfert semi-sec à l'aide de l'appareil « Vacugene XL Vacuum blotting System » (Pharmacia) selon les recommandations du fabricant. L'ADN transféré sur le filtre nylon est fixé aux UV pendant 3 min à 312 nm. Le filtre est ensuite pré hybridé pendant 2 heures à 42°C dans du tampon « DIG easy hyb » (Roche ref: 1585738), puis hybridé une nuit à 42 °C dans du tampon « DIG easy hyb » contenant 20-50 ng d'une sonde spécifique PvuII marquée à la digoxygénine/ml de tampon. Cette sonde reconnaît spécifiquement le locus *cap d'Haemophilus influenzae sérotype b.* Le filtre est ensuite lavé deux fois à l'aide d'un tampon de faible « stringeance » à 65°C suivi d'un lavage dans un tampon de forte « stringeance ». Le filtre est ensuite révélé à l'aide d'un substrat luminescent (CDP -star : Roche Réf: 2041677) après avoir ajouté une solution d'anticorps antidigoxygenine marquée à la phopshatase alcaline en utilisant le kit « Dig-Chem-link labeling and detection Set » (Roche). Le profil électrophorétique obtenu est représenté à la figure 3. On observe deux bandes de 18 Kb et de 45 Kb, ce qui indique que la structure du locus *cap* de la population mère est hétérogène. Une partie de la population possède un locus *cap* qui contient deux copies du gène de 18 Kb, correspondant à la bande électrophorétique de 45 Kb tandis que l'autre partie possède un locus *cap* sous une forme non dupliquée, correspondant à la bande électrophorétique de 18 Kb. Par conséquent, la population mère est un mélange de bactéries capsulées et non capsulées. Cette hétérogénéité est par ailleurs confirmée en utilisant le test de détermination du pourcentage de colonies blanches que l'on obtient après ensemencement de la population mère sur un milieu solide sélectif (voir exemple 2).

3.2) Culture de la population mère sur milieu solide sélectif: Détermination du pourcentage de bactéries formant des colonies blanches sur milieu solide sélectif et dérivation d'une population fille constituée essentiellement de bactéries capsulées.

### 3.2.1) Détermination du pourcentage de bactéries formant des colonies blanches

L'étape de culture en milieu solide sélectif et l'analyse morphologique des colonies obtenues sont réalisées selon les mêmes conditions opératoires décrites dans l'exemple 2. La composition du milieu solide sélectif correspond à celle du milieu sélectif A de l'exemple 2.

On détermine le nombre de colonies blanches pour 100 colonies visualisées. 60% des colonies sont sous forme de colonies blanches ce qui confirme bien que la population mère initiale est hétérogène et renferme un mélange de bactéries capsulées et non capsulées.

### 3.2.2) Sélection et caractérisation de la population fille

### 3.2.2.1) Sélection de la population fille

On repique une colonie blanche qui a été obtenue au bout de 18 à 24 heures de culture sur le milieu solide sélectif A dans un tube contenant 2 ml d'une composition de milieu liquide identique au milieu solide sélectif sans Bacto agar. Après une nouvelle incubation de 20 heures à 37°C sous agitation, le contenu du tube est transféré dans un erlen contenant 50ml d'un milieu liquide selon l'invention dont la composition par litre est la suivante :
β-NAD: 5 mg
protoporphyrine IX: 1 mg
glucose : 20 g
extrait de levure : 5 g
peptone de pois (Hypea 7404 (Quest)) :7,42 g
lactate de sodium en solution aqueuse à 60% : 1,49 ml
cystine: 0,07 g
tryptophane: 0,02 g
Na₂HPO₄, 12H₂O: 31,14 g
NaH₂PO₄, 2H₂O: 2,03 g
(NH₄)₂SO₄: 1 g
MgSO₄, 7H₂O : 0,4 g
CaCl₂, 2H₂O : 0,02 g

L'erlen est placé à l'étuve à 37°C sous agitation. Lorsque la D.O. à 600 nm est voisine de 2, on rajoute un volume de glycérol tel que sa concentration finale dans la suspension bactérienne soit de 20% (V/V). La suspension bactérienne est répartie en tubes Nunc sous 1 ml avant d'être congelée à -70°C. On a ainsi dérivé une population bactérienne fille sous forme de congelât produite à partir d'une colonie blanche obtenue sur une composition de milieu solide sélectif et qui est issue d'une bactérie de la population mère.

### 3.2.2.2) Caractérisation de la population bactérienne fille

L'analyse du locus *cap* de la population fille a été effectuée selon le protocole décrit dans le paragraphe 3.1.1.2. Le profil électrophorétique montre une seule bande de 45 Kb ce qui indique que le locus *cap* de la population fille est essentiellement sous une forme dupliquée du gêne de 18 Kb (cf. figure 3). Par conséquent, la population bactérienne fille est essentiellement constituée de bactéries capsulées. L'homogénéité de cette population est confirmée par le fait qu'elle produit également 100% de colonies blanches lorsqu'elle est ensemencée sur un milieu solide sélectif.

### 3.3) Comparaison de la production en PRP par les bactéries de la population mère et de la population fille

On inocule directement le contenu d'une ampoule contenant ≈ 10¹⁰ bactéries provenant soit de la population mère, soit de la population fille dans un erlen contenant 200 ml d'un milieu liquide dont la composition est celle indiquée dans le paragraphe 3.2.2.1.

Après une incubation de 24 h à 37°C + /- 1°C, sous agitation (175 rpm), on prélève le surnageant de culture, puis on détermine la concentration en PRP par ELISA selon la méthode décrite dans l'exemple 1. Le même essai a été répété 3 fois. Les résultats figurent dans le tableau ci-dessous. Les valeurs indiquées représentent la valeur moyenne des 3 essais.

| | Essai 1 | Essai 2 | Essai 3 |
|---|---|---|---|
| Congelât mère | 145 * | 185 | 116 |
| Population hétérogène | | | |
| Congelât fille | 402 | 447 | 429 |
| Population homogène de bactéries capsulées | | | |

| | | | |
|---|---|---|---|
| *: résultats exprimés en mg/l | | | |

Conclusion : la production en PRP de la population fille constituée d'une population homogène de bactéries capsulées est améliorée ≈ d'un facteur 3. Par conséquent le procédé qui consiste à utiliser une étape de culture sur un milieu solide sélectif qui permet de sélectionner des colonies blanches renfermant 100% de bactéries capsulées, améliore les rendements en PRP obtenus. Ce procédé peut être également utilisé pour constituer une population de bactéries entièrement capsulées à partir d'une population initiale qui contient un mélange de bactéries capsulées et non capsulées.

### Exemple 4 : Rôle du milieu de culture stabilisant sur la population bactérienne et sur la production de PRP

La population bactérienne de départ est constituée d'une population de bactéries entièrement capsulées dont le locus *cap* contient au moins deux copies du gène de 18 Kb et qui produit 100% de colonies blanches sur un milieu solide sélectif.

### 4.1) Protocole opératoire

On inocule le contenu d'un congelât contenant par ml de 10⁸ à 10¹⁰ bactéries provenant de la population fille sélectionnée selon le protocole opératoire du paragraphe 3.2.2.1 dans un fermenteur de 1 litre contenant 500 ml d'un milieu liquide dont la composition est celle indiquée dans le paragraphe 3.2.2.1. Après une incubation de 14 heures à 37°C sous agitation, on transfère un volume de la première culture dans un deuxième fermenteur de 1 litre contenant 500 ml du même milieu liquide de manière à avoir une D.O. initiale égale à 0,3. Après une nouvelle incubation de ∼ 5 heures dans les mêmes conditions (D.O. obtenue voisine de 4), on transfère un volume de la deuxième culture dans un troisième fermenteur de 1 litre contenant 500 ml de milieu de manière à avoir une DO initiale égale à 0,3 puis après une incubation de ≈ 3 heures (D.O. obtenue voisine de 4), on verse le volume dans un quatrième fermenteur de 1 litre contenant 500 ml du même milieu liquide. Ce protocole opératoire est une transposition à l'échelle du laboratoire des étapes qui sont normalement effectuées pour la production industrielle de PRP dans un fermenteur de 13 000 litres.

On a calculé le nombre de générations bactériennes à la fin de chaque culture en employant la formule classique N= Log X/X0 x 1/Log 2 dans lequel X représente la biomasse à la fin de la culture et X0 la biomasse au début de la culture. Le nombre de générations bactériennes cumulées obtenues à la fin de la quatrième culture en fermenteur de 1 litre correspond en fait au nombre de générations bactériennes obtenues à la fin de la culture dans un fermenteur de 13 000 litres. A la fin de chaque culture, on a caractérisé le locus *cap* et on a déterminé le pourcentage de bactéries qui forment des colonies blanches sur milieu solide sélectif selon les méthodes décrites dans l'exemple 3. Les résultats obtenus sont regroupés dans le tableau ci dessous

| | Population de départ | Culture 1 | Culture 2 | Culture 3 | Culture 4 |
|---|---|---|---|---|---|
| Nombre de générations | | 11,46 | 3,69 | 3,75 | 5,19 |
| Locus *cap* | Pas de forme non dupliquée visible à l'électrophorèse | Pas de forme non dupliquée visible à l'électrophorèse | Pas de forme non dupliquée visible à l'électrophorèse | Pas de forme non dupliquée visible à l'électrophorèse | Pas de forme non dupliquée visible à l'électrophorèse |
| % de colonies blanches | 100 | 97 | 98 | 99 | 100 |
| PRP (mg/l) | | 842 | 904 | 719 | 782 |

Le nombre de générations cumulées à l'issue de la quatrième culture est de 24,09 générations.

Les cultures successives ne modifient pas les caractéristiques de la population bactérienne qui reste entièrement capsulée au cours des cultures successives. La production de PRP reste également stable tout au long de la culture à un niveau très élevé. La composition de ce milieu exerce donc un rôle stabilisant sur la population bactérienne capsulée puisque les caractéristiques de la population bactérienne ne se modifient pas de façon sensible au cours de la culture.

## Revendications

1. Un milieu pour la culture d'*Haemophilus influenzae serotype b,* **caractérisé en ce que** la source d'azote protéique est d'origine non animale et comprend au moins une peptone végétale et **en ce que** la source d'hème consiste en de la protoporphyrine IX.

2. Un milieu selon la revendication 1, dans lequel la concentration en protoporphyrine IX est d'au moins 0,01 mg/l.

3. Un milieu selon la revendication 2, dans lequel la concentration en protoporphyrine IX est entre 0,1 mg/l et 5mg/l.

4. Un milieu selon l'une des revendications 1 à 3, dans lequel la peptone végétale est une peptone de blé.

5. Un milieu selon l'une des revendications 1 à 4, dans lequel la peptone végétale est une peptone de petit pois.

6. Un milieu selon l'une des revendications 1 à 5, dans lequel la concentration totale en peptone végétale dans le milieu de culture équivaut à une concentration en azote protéique comprise entre 0,08 g/l et 2,25 g/l.

7. Un milieu selon l'une des revendications 1 à 6, exempt de tout contaminant d'origine animale.

8. Un milieu selon l'une des revendications 5 à 7 **caractérisé en ce que** ledit milieu est liquide et comprend :
- de 0,1 mg/l à 5 mg/l de protoporphyrine IX,
- de 2 à 50 mg/l de β NAD,
- de 2 à 20 g/l de glucose,
- de 2 à 5 g/l d'un extrait de levure,
- une peptone de petit pois équivalent à une concentration en azote protéique comprise entre 0,4g/l et 1,5g/ et
- un cocktail d'ions minéraux comprenant les ions Na⁺, NH4 ⁺, Ca⁺⁺, Mg⁺⁺, HPO₄⁻⁻, H₂PO4⁻, SO₄⁻⁻, et Cl⁻ sous forme de solutions salines de sorte que le pH du milieu se situe entre 6,5 et 7,5, de façon préférée entre 7,0 et 7,5.

9. Un procédé de production du polyribosylribitol phosphate (PRP) dans lequel :
(i) on cultive *Haemophilus influenzae de sérotype b* dans un milieu de culture selon l'une des revendications 1 à 8,
(ii) on prélève le surnageant de la culture obtenue en (i), et
(iii) on extrait le PRP du surnageant de culture.

10. Un procédé de production du polyribosylribitol phosphate (PRP) dans lequel :
(i) on cultive *Haemophilus influenzae de sérotype b* sur un milieu de culture solide,
(ii) on transfère et on cultive une ou plusieurs colonies obtenues en (i) dans un milieu de culture selon l'une des revendications 1 à 8,
(iii) on prélève le surnageant de la culture obtenue en (ii), et
(iv) on extrait le PRP du surnageant de culture.

11. Un procédé selon la revendication 10, dans lequel la source d'azote protéique du milieu de culture solide est d'origine non animale et comprend au moins une peptone végétale.

12. Un procédé selon la revendication 11, dans lequel la peptone végétale du milieu de culture solide est une peptone de petit pois.

13. Un procédé selon la revendication 11 ou 12, dans lequel la source d'hème du milieu de culture solide consiste en de la protoporphyrine IX.

14. Un procédé selon l'une des revendications 10 à 13, dans lequel le milieu solide comprend :
- au moins 1 mg/l de β NAD,
- au moins 0,5 mg/l de protoporphyrine IX,
- au moins une peptone végétale et un extrait de levure en quantité suffisante pour que la concentration en azote protéique dans le milieu solide soit d'au moins 0,2 g/l et dans une proportion telle que le ratio entre la quantité de peptone végétale et la quantité d'extrait de levure dans le milieu soit de 0,1 à 9 lorsque la concentration en azote protéique du milieu est de 0,2 g/l à 0,8 g/l et qu'il soit de 1 à 9 lorsque la concentration en azote protéique du milieu est > 0,8 g/l,
- un carbohydrate,
- un agent détoxifiant, et
- un cocktail d'ions minéraux comprenant les ions Na⁺, K⁺, Ca⁺⁺, Mg⁺⁺, Fe⁺⁺⁺, HPO₄⁻⁻, H₂PO4⁻, SO₄⁻⁻, et Cl⁻ sous forme de solutions salines de sorte que le pH du milieu se situe entre 6,5 et 7,5, de façon préférée entre 7,0 et 7,5.

15. Un procédé selon la revendication 14, dans lequel le milieu solide comprend :
- de 5 à 50 mg/l de β NAD,
- de 0,5 à 5 mg/l de protoporphyrine IX,
- de 1 à 10 g/l de glucose,
- de 1 à 10 mg/l de Tween 80,
- de 3 à 4 g/l de K₂HPO₄,
- de 0,9 à 3 g/l de KH₂PO₄,
- de 0,5 à 2 g/l de K₂SO₄,
- de 20 à 500 mg/l de MgCl₂,
- de 2 à 50 mg/l de CaCl₂, 2H₂O,
- de 1 à 5 mg/l de FeCl₃, 6H₂O,
- de 4 à 8 g/l de NaCl,
- de 4 à 8 g/l d'un extrait de levure, et
- de 4 à 8 g/l d'une peptone de petit pois de telle sorte que le ratio entre la quantité de peptone de petit pois et la quantité d'extrait de levure soit ≥ 1 lorsque la concentration en azote protéique du milieu est > 0,8 g/l.

16. Un procédé selon la revendication 14 ou 15, dans lequel on transfère dans le milieu de culture liquide uniquement les colonies blanches.

17. Un procédé selon l'une des revendications 10 à 16, dans lequel le milieu de culture solide et le milieu de culture liquide sont dépourvus de tout contaminant d'origine animale.

18. Un procédé de production d'une population de bactéries entièrement capsulées d'*Haemophilus influenzae de sérotype b* dans lequel :
(i) on cultive *Haemophilus influenzae de sérotype b* sur un milieu solide comprenant :
- de 5 à 50 mg/l de β NAD,
- de 0,5 à 5 mg/l de protoporphyrine IX,
- de 1 à 10 g/l de glucose,
- de 1 à 10 mg/l de Tween 80,
- de 3 à 4 g/l de K₂HPO₄,
- de 0,9 à 3 g/l de KH₂PO₄,
- de 0,5 à 2 g/l de K₂SO₄,
- de 20 à 500 mg/l de MgCl₂,
- de 2 à 50 mg/l de CaCl₂, 2H₂O,
- de 1 à 5 mg/l de FeCl₃, 6H₂O,
- de 4 à 8 g/l de NaCl,
- de 4 à 8 g/l d'un extrait de levure et,
- de 4 à 8 g/l d'une peptone de petit pois de telle sorte que le ratio entre la quantité de peptone de petit pois et la quantité d'extrait de levure soit ≥ 1 lorsque la concentration en azote protéique du milieu est > 0,8g/l ;
(ii) on transfère et on cultive une ou plusieurs colonies blanches obtenues en (i) dans un milieu de culture liquide comprenant:
- de 0,1 à 5 mg/l de protoporphyrine IX,
- de 2 à 50 mg/l de β NAD,
- de 2 à 20 g/l de glucose,
- de 2 à 5 g/l d'un extrait de levure,
- une peptone de petit pois équivalent à une concentration en azote protéique de 0,4 g/l à 1,5 g/l, et
- un cocktail d'ions minéraux comprenant les ions Na⁺, NH4⁺, Ca⁺⁺, Mg⁺⁺, HPO₄⁻⁻, H₂PO4⁻, SO₄⁻⁻, et Cl⁻ sous forme de solutions salines de sorte que le pH du milieu se situe entre 6,5 et 7,5, de façon préférée entre 7,0 et 7,5 ; et
(iii) on congèle ou on lyophilise la population bactérienne obtenue en (ii).

19. Un procédé selon la revendication 18, dans lequel toutes les étapes sont réalisées au moyen de milieux dépourvus de tout contaminant d'origine animale.

20. Un milieu de culture solide d'*Haemophilus influenzae serotype b,* dont la source d'azote protéique est d'origine non animale et qui comprend:
- au moins 1 mg/l de β NAD,
- au moins 0,5 mg/l de protoporphyrine IX,
- une peptone végétale et un extrait de levure en quantité suffisante pour que la concentration en azote protéique dans le milieu soit d'au moins 0,2 g/l d'azote protéique et dans une proportion telle que le ratio entre la quantité de peptone végétale et la quantité d'extrait de levure dans le milieu soit de 0,1 à 9 lorsque la concentration en azote protéique du milieu est de 0,2 g/l à 0,8 g/l et qu'il soit de 1 à 9 lorsque la concentration en azote protéique du milieu est > 0,8 g/l,
- un carbohydrate,
- un agent détoxifiant, et
- un cocktail d'ions minéraux comprenant les ions Na⁺, K⁺, Ca⁺⁺, Mg⁺⁺, Fe⁺⁺⁺, HPO₄⁻⁻, H₂PO4⁻, SO₄⁻⁻, et Cl⁻ sous forme de solutions salines de sorte que le pH du milieu se situe entre 6,5 et 7,5, de préférence entre 7,0 et 7,5.

21. Un milieu solide selon la revendication 20 qui comprend :
- de 5 à 50 mg/l de β NAD,
- de 0,5 à 5 mg/l de protoporphyrine IX,
- de 1 à 10 g/l de glucose,
- de 1 à 10 mg/l de Tween 80,
- de 3 à 4 g/l de K₂HPO₄,
- de 0,9 à 3 g/l de KH₂PO₄,
- de 0,5 à 2 g/l de K₂SO₄,
- de 20 à 500 mg/l de MgCl₂,
- de 2 à 50 mg/l de CaCl₂, 2H₂O,
- de 1 à 5 mg/l de FeCl₃, 6H₂O,
- de 4 à 8 g/l de NaCl,
- de 4 à 8 g/l d'un extrait de levure, et
- de 4 à 8 g/l d'une peptone de petit pois de telle sorte que le ratio entre la quantité de peptone de petit pois et la quantité d'extrait de levure soit ≥ 1 lorsque la concentration en azote protéique du milieu est > 0,8 g/l.

## Patentansprüche

1. Medium für die Kultur von *Haemophilus influenzae Serotyp b,* **dadurch gekennzeichnet, dass** die Protein-Stickstoffquelle nicht-tierischen Ursprungs ist und mindestens ein pflanzliches Pepton umfasst, und dadurch, dass die Quelle von Häm aus Protoporphyrin IX besteht.

2. Medium nach Anspruch 1, wobei die Konzentration an Protoporphyrin IX mindestens 0,01 mg/l beträgt.

3. Medium nach Anspruch 2, wobei die Konzentration an Protoporphyrin IX zwischen 0,1 mg/l und 5 mg/l beträgt.

4. Medium nach einem der Ansprüche 1 bis 3, wobei es sich bei dem pflanzlichen Pepton um ein Weizenpepton handelt.

5. Medium nach einem der Ansprüche 1 bis 4, wobei es sich bei dem pflanzlichen Pepton um ein Erbsenpepton handelt.

6. Medium nach einem der Ansprüche 1 bis 5, wobei die Gesamtkonzentration an pflanzlichem Pepton in dem Kulturmedium einer Protein-Stickstoffkonzentration zwischen 0,08 g/l und 2,25 g/l entspricht.

7. Medium nach einem der Ansprüche 1 bis 6, das von jeglicher Verunreinigung tierischen Ursprungs frei ist.

8. Medium nach einem der Ansprüche 5 bis 7, **dadurch gekennzeichnet, dass** das Medium flüssig ist und Folgendes umfasst:
- 0,1 mg/l bis 5 mg/l Protoporphyrin IX,
- 2 bis 50 mg/l β-NAD,
- 2 bis 20 g/l Glucose,
- 2 bis 5 g/l eines Hefeextrakts,
- ein Erbsenpepton entsprechend einer Protein-Stickstoffkonzentration zwischen 0,4 g/l und 1,5 g/l und
- einen Cocktail anorganischer Ionen, der die Ionen Na⁺, NH4⁺, Ca⁺⁺, Mg⁺⁺, BPO4⁻⁻, H2PO4⁻, SO4⁻⁻ und Cl⁻ in Form von Salzlösungen derart umfasst, dass der pH des Mediums zwischen 6,5 und 7,5, bevorzugt zwischen 7,0 und 7,5, liegt.

9. Verfahren zur Produktion von Polyribosylribitphosphat (PRP), wobei:
(i) man *Haemophilus influenzae Serotyp b* in einem Kulturmedium nach einem der Ansprüche 1 bis 8 kultiviert,
(ii) den Überstand der in (i) erhaltenen Kultur entnimmt und
(iii) PRP aus dem Kulturüberstand extrahiert.

10. Verfahren zur Produktion von Polyribosylribitphosphat (PRP), wobei:
(i) man *Haemophilus influenzae Serotyp b* auf einem festen Kulturmedium kultiviert,
(ii) eine oder mehrere in (i) erhaltene Kolonien in ein Kulturmedium nach einem der Ansprüche 1 bis 8 überführt und kultiviert,
(iii) den Überstand der in (ii) erhaltenen Kultur entnimmt und
(iv) PRP aus dem Kulturüberstand extrahiert.

11. Verfahren nach Anspruch 10, wobei die Protein-Stickstoffquelle des festen Kulturmediums nichttierischen Ursprungs ist und mindestens ein pflanzliches Pepton enthält.

12. Verfahren nach Anspruch 11, wobei es sich bei dem pflanzlichen Pepton des festen Kulturmediums um ein Erbsenpepton handelt.

13. Verfahren nach Anspruch 11 oder 12, wobei die Quelle von Häm des festen Kulturmediums aus Protoporphyrin IX besteht.

14. Verfahren nach einem der Ansprüche 10 bis 13, wobei das feste Medium Folgendes umfasst:
- mindestens 1 mg/l β-NAD,
- mindestens 0,5 mg/l Protoporphyrin IX,
- mindestens ein pflanzliches Pepton und einen Hefeextrakt in einer ausreichenden Menge, dass die Protein-Stickstoffkonzentration in dem festen Medium mindestens 0,2 g/l beträgt, und in einem derartigen Anteil, dass das Verhältnis zwischen der Menge an pflanzlichem Pepton und der Menge an Hefeextrakt in dem Medium von 0,1 bis 9 reicht, wenn die Protein-Stickstoffkonzentration des Mediums 0,2 g/l bis 0,8 g/l beträgt, und dass es von 1 bis 9 reicht, wenn die Protein-Stickstoffkonzentration des Mediums > 0,8 g/l ist,
- ein Kohlenhydrat,
- ein Detoxifikans und
- einen Cocktail anorganischer Ionen, der die Ionen Na⁺, K⁺, Ca⁺⁺, Mg⁺⁺, Fe⁺⁺⁺, HPO4⁻⁻, H2PO4⁻, SO4⁻⁻ und Cl⁻ in Form von Salzlösungen derart umfasst, dass der pH des Mediums zwischen 6,5 und 7,5, bevorzugt zwischen 7,0 und 7,5, liegt.

15. Verfahren nach Anspruch 14, wobei das feste Medium Folgendes umfasst:
- 5 bis 50 mg/l β-NAD,
- 0,5 bis 5 mg/l Protoporphyrin IX,
- 1 bis 10 g/l Glucose,
- 1 bis 10 mg/l Tween 80,
- 3 bis 4 g/l K₂HPO₄,
- 0,9 bis 3 g/l KH₂PO₄,
- 0,5 bis 2 g/l K₂SO₄,
- 20 bis 500 mg/l MgCl₂,
- 2 bis 50 mg/l CaCl₂, 2H₂O,
- 1 bis 5 mg/l FeCl₃, 6H₂O,
- 4 bis 8 g/l NaCl,
- 4 bis 8 g/l eines Hefeextrakts und
- 4 bis 8 g/l eines Erbsenpeptons, derart, dass das Verhältnis zwischen der Menge an Erbsenpepton und der Menge an Hefeextrakt ≥ 1 ist, wenn die Protein-Stickstoffkonzentration des Mediums > 0,8 g/l ist.

16. Verfahren nach Anspruch 14 oder 15, wobei man in das flüssige Kulturmedium nur weiße Kolonien überführt.

17. Verfahren nach einem der Ansprüche 10 bis 16, wobei das feste Kulturmedium und das flüssige Kulturmedium von jeglicher Verunreinigung tierischen Ursprungs frei sind.

18. Verfahren zur Produktion einer vollständig eingekapselten Bakterienpopulation von *Haemophilus influenzae Serotyp b,* wobei:
(i) man *Haemophilus influenzae Serotyp b* auf einem festen Medium kultiviert, umfassend:
- 5 bis 50 mg/l β-NAD,
- 0,5 bis 5 mg/l Protoporphyrin IX,
- 1 bis 10 g/l Glucose,
- 1 bis 10 mg/l Tween 80,
- 3 bis 4 g/l K₂HPO₄,
- 0,9 bis 3 g/l KH₂PO₄,
- 0,5 bis 2 g/l K₂SO₄,
- 20 bis 500 mg/l MgCl₂,
- 2 bis 50 mg/l CaCl₂, 2H₂O,
- 1 bis 5 mg/l FeCl₃, 6H₂O,
- 4 bis 8 g/l NaCl
- 4 bis 8 g/l eines Hefeextrakts und
- 4 und 8 g/l eines Erbsenpeptons, derart, dass das Verhältnis zwischen der Menge an Erbsenpepton und der Menge an Hefeextrakt ≥ 1 ist, wenn die Protein-Stickstoffkonzentration des Mediums > 0,8 g/l ist,
(ii) man eine oder mehrere in (i) erhaltene weiße Kolonien in ein flüssiges Kulturmedium überführt und darin kultiviert, das Folgendes umfasst:
- 0,1 bis 5 mg/l Protoporphyrin IX,
- 2 bis 50 mg/l β-NAD,
- 2 bis 20 g/l Glucose,
- 2 bis 5 g/l eines Hefeextrakts,
- ein Erbsenpepton entsprechend einer Protein-Stickstoffkonzentration von 0,4 g/l bis 1,5 g/l und
- einen Cocktail anorganischer Ionen, der die Ionen Na⁺, NH4⁺, Ca⁺⁺, Mg⁺⁺, BPO4⁻⁻, H2PO4⁻, SO4⁻⁻ und Cl⁻ in Form von Salzlösungen derart umfasst, dass der pH des Mediums zwischen 6,5 und 7,5, bevorzugt zwischen 7,0 und 7,5, liegt,
und
(iii) man die in (ii) erhaltene Bakterienpopulation einfriert oder lyophilisiert.

19. Verfahren nach Anspruch 18, wobei alle Schritte mithilfe von Medien durchgeführt werden, die von jeglicher Verunreinigung tierischen Ursprungs frei sind.

20. Festes Kulturmedium für *Haemophilus influenzae Serotyp b,* dessen Quelle für Protein-Stickstoff nichttierischen Ursprungs ist und das Folgendes umfasst:
- mindestens 1 mg/l β-NAD,
- mindestens 0,5 mg/l Protoporphyrin IX,
- ein pflanzliches Pepton und einen Hefeextrakt in einer ausreichenden Menge, dass die Protein-Stickstoffkonzentration in dem Medium mindestens 0,2 g/l Protein-Stickstoff beträgt, und in einem derartigen Anteil, dass das Verhältnis zwischen der Menge an pflanzlichem Pepton und der Menge an Hefeextrakt in dem Medium von 0,1 bis 9 reicht, wenn die Protein-Stickstoffkonzentration des Mediums 0,2 g/l bis 0,8 g/l beträgt, und dass es von 1 bis 9 reicht, wenn die Protein-Stickstoffkonzentration des Mediums > 0,8 g/l ist,
- ein Kohlenhydrat,
- einem Detoxifikans und
- einen Cocktail anorganischer Ionen, der die Ionen Na⁺, K⁺, Ca⁺⁺, Mg⁺⁺, Fe⁺⁺⁺, HPO4⁻⁻, H2PO4⁻, SO4⁻⁻ und Cl⁻ in Form von Salzlösungen derart umfasst, dass der pH des Mediums zwischen 6,5 und 7,5, bevorzugt zwischen 7,0 und 7,5, liegt.

21. Festes Medium nach Anspruch 20, das Folgendes umfasst:
- 5 bis 50 mg/l β-NAD,
- 0,5 bis 5 mg/l Protoporphyrin IX,
- 1 bis 10 g/l Glucose,
- 1 bis 10 mg/l Tween 80,
- 3 bis 4 g/l K₂HPO₄,
- 0,9 bis 3 g/l KH₂PO₄,
- 0,5 bis 2 g/l K₂SO₄,
- 20 bis 500 mg/l MgCl₂,
- 2 bis 50 mg/l CaCl₂, 2H₂O,
- 1 bis 5 mg/l FeCl₃, 6H₂O,
- 4 bis 8 g/l NaCl,
- 4 bis 8 g/l eines Hefeextrakts und
- 4 bis 8 g/l eines Erbsenpeptons, derart, dass das Verhältnis zwischen der Menge an Erbsenpepton und der Menge an Hefeextrakt ≥ 1 ist, wenn die Protein-Stickstoffkonzentration des Mediums > 0,8 g/l ist.

## Claims

1. Medium for the culture of *Haemophilus influenzae serotype b,* **characterized in that** the source of protein nitrogen is of nonanimal origin and comprises at least one plant peptone and wherein the heme source consists of protoporphyrin IX.

2. Medium according to Claim 1, in which the protoporphyrin IX concentration is at least 0.01 mg/l.

3. Medium according to Claim 2, in which the protoporphyrin IX concentration is between 0.1 mg/l and 5 mg/l.

4. Medium according to one of Claims 1 to 3, in which the plant peptone is a wheat peptone.

5. Medium according to one of Claims 1 to 4, in which the plant peptone is a garden pea peptone.

6. Medium according to one of Claims 1 to 5, in which the total plant peptone concentration in the culture medium is equivalent to a protein nitrogen concentration of between 0.08 g/l and 2.25 g/l.

7. Medium according to one of Claims 1 to 6, free of any contaminant of animal origin.

8. Medium according to one of Claims 5 to 7, **characterized in that** said medium is liquid and comprises:
- from 0.1 mg/l to 5 mg/l of protoporphyrin IX,
- from 2 to 50 mg/l of β-NAD,
- from 2 to 20 g/l of glucose,
- from 2 to 5 g/l of a yeast extract,
- a garden pea peptone equivalent to a protein nitrogen concentration of between 0.4 g/l and 1.5 g/ and
- a cocktail of inorganic ions comprising Na⁺, NH₄⁺, Ca⁺⁺, Mg⁺⁺, HPO₄⁻⁻, H₂PO₄⁻, SO₄⁻⁻ and Cl⁻ ions, in the form of salt solutions such that the pH of the medium is between 6.5 and 7.5, preferably between 7.0 and 7.5.

9. Method for producing polyribosyl ribitol phosphate (PRP) in which:
(i) *Haemophilus influenzae serotype b* is cultured in a culture medium according to one of Claims 1 to 8,
(ii) the culture supernatant obtained in
(i) is collected, and
(iii) the PRP is extracted from the culture supernatant.

10. Method for producing polyribosyl ribitol phosphate (PRP) in which:
(i) *Haemophilus influenzae serotype b* is cultured on a solid culture medium,
(ii) one or more colonies obtained in (i) are transferred into and cultured in a culture medium according to one of Claims 1 to 8,
(iii) the culture supernatant obtained in
(ii) is collected, and
(iv) the PRP is extracted from the culture supernatant.

11. Method according to Claim 10, in which the source of protein nitrogen of the solid culture medium is of nonanimal origin and comprises at least one plant peptone.

12. Method according to Claim 11, in which the plant peptone of the solid culture medium is a garden pea peptone.

13. Method according to Claim 11 or 12, in which the heme source of the solid culture medium consists of protoporphyrin IX.

14. Method according to one of Claims 10 to 13, in which the solid medium comprises:
- at least 1 mg/l of β-NAD,
- at least 0.5 mg/l of protoporphyrin IX,
- at least one plant peptone and a yeast extract in a sufficient quantity for the protein nitrogen concentration in the solid medium to be at least 0.2 g/l and in a proportion such that the ratio between the quantity of plant protein and the quantity of yeast extract in the medium is 0.1 to 9 when the concentration of protein nitrogen of the medium is 0.2 g/l to 0.8 g/l and is 1 to 9 when the concentration of protein nitrogen of the medium is > 0.8 g/l,
- a carbohydrate,
- a detoxifying agent, and
- a cocktail of inorganic ions comprising Na⁺, K⁺, Ca⁺⁺, Mg⁺⁺, Fe⁺⁺⁺, HPO₄⁻⁻, H₂PO4⁻, SO₄⁻⁻ and Cl⁻ ions in the form of salt solutions such that the pH of the medium is between 6.5 and 7.5, preferably between 7.0 and 7.5.

15. Method according to Claim 14, in which the solid medium comprises:
- from 5 to 50 mg/l of β-NAD,
- from 0.5 to 5 mg/l of protoporphyrin IX,
- from 1 to 10 g/l of glucose,
- from 1 to 10 mg/l of Tween 80,
- from 3 to 4 g/l of K₂HPO₄,
- from 0.9 to 3 g/l of KH₂PO₄,
- from 0.5 to 2 g/ml of K₂SO₄,
- from 20 to 500 mg/l of MgCl₂,
- from 2 to 50 mg/l of CaCl₂·2H₂O,
- from 1 to 5 mg/l of FeCl₃·6H₂O,
- from 4 to 8 g/l of NaCl,
- from 4 to 8 g/l of a yeast extract, and
- from 4 to 8 g/l of a garden pea peptone such that the ratio between the quantity of garden pea peptone and the quantity of yeast extract is ≥ 1 when the protein nitrogen concentration of the medium is > 0.8 g/l.

16. Method according to Claim 14 or 15, in which only white colonies are transferred into the liquid culture medium.

17. Method according to one of Claims 10 to 16, in which the solid culture medium and the liquid culture medium are free of any contaminant of animal origin.

18. Method for producing a population of completely capsulated *Haemophilus influenzae serotype b* bacteria in which:
(i) *Haemophilus influenzae serotype b* is cultured on a solid medium comprising:
- from 5 to 50 mg/l of β-NAD,
- from 0.5 to 5 mg/l of protoporphyrin IX,
- from 1 to 10 g/l of glucose,
- from 1 to 10 mg/l of Tween 80,
- from 3 to 4 g/l of K₂HPO₄,
- from 0.9 to 3 g/l of KH₂PO₄,
- from 0.5 to 2 g/l of K₂SO₄,
- from 20 to 500 mg/l of MgCl₂,
- from 2 to 50 mg/l of CaCl₂·2H₂O,
- from 1 to 5 mg/l of FeCl₂, ·6H₂O,
- from 4 to 8 g/l of NaCl,
- from 4 to 8 g/l of a yeast extract, and
- from 4 to 8 g/l of a garden pea peptone such that the ratio between the quantity of garden pea peptone and the quantity of yeast extract is ≥ 1 when the protein nitrogen concentration of the medium is > 0.8 g/l;
(ii) one or more white colonies obtained in
(i) are transferred into and cultured in a liquid culture medium comprising:
- from 0.1 to 5 mg/l of protoporphyrin IX,
- from 2 to 50 mg/l of β-NAD,
- from 2 to 20 g/l of glucose,
- from 2 to 5 g/l of a yeast extract,
- a garden pea peptone equivalent to a protein nitrogen concentration of 0.4 g/l to 1.5 g/l, and
- a cocktail of inorganic ions comprising Na⁺, NH₄⁺, Ca⁺⁺, Mg⁺⁺, HPO₄⁻⁻, H₂PO₄⁻, SO₄⁻⁻ and Cl⁻ ions in the form of salt solutions such that the pH of the medium is between 6.5 and 7.5, preferably between 7.0 and 7.5; and (iii) the bacterial population obtained in
(ii) is frozen or freeze-dried.

19. Method according to Claim 18, in which all the stages are carried out using media that are free of any contaminant of animal origin.

20. Solid culture medium for *Haemophilus influenzae serotype b,* the source of protein nitrogen for which is of nonanimal origin and which comprises:
- at least 1 mg/l of β-NAD,
- at least 0.5 mg/l of protoporphyrin IX,
- a plant peptone and a yeast extract in a sufficient quantity for the protein nitrogen concentration in the medium to be at least 0.2 g/l of protein nitrogen and in a proportion such that the ratio between the quantity of plant peptone and the quantity of yeast extract in the medium is 0.1 to 9 when the protein nitrogen concentration of the medium is 0.2 g/l to 0.8 g/l and is 1 to 9 when the protein nitrogen concentration of the medium is > 0.8 g/l,
- a carbohydrate,
- a detoxifying agent, and
- a cocktail of inorganic ions comprising Na⁺, K⁺, Ca⁺⁺, Mg⁺⁺, Fe⁺⁺⁺, HPO₄⁻⁻", H₂PO4⁻, SO₄⁻⁻ and Cl⁻ in the form of salt solutions such that the pH of the medium is between 6.5 and 7.5, preferably between 7.0 and 7.5.

21. Solid medium according to Claim 20, which comprises:
- from 5 to 50 mg/l of β-NAD,
- from 0.5 to 5 mg/l of protoporphyrin IX,
- from 1 to 10 g/l of glucose,
- from 1 to 10 mg/l of Tween 80,
- from 3 to 4 g/l of K₂HPO₄,
- from 0.9 to 3 g/l of KH₂PO₄,
- from 0.5 to 2 g/l of K₂SO₄,
- from 20 to 500 mg/l of MgCl₂,
- from 2 to 50 mg/l of CaCl₂·2H₂O,
- from 1 to 5 mg/l of FeCl₃·6H₂O,
- from 4 to 8 g/l of NaCl,
- from 4 to 8 g/l of a yeast extract, and
- from 4 to 8 g/l of a garden pea peptone such that the ratio between the quantity of garden pea peptone and the quantity of yeast extract is ≥ 1 when the protein nitrogen concentration of the medium is > 0.8 g/l.
